# EUROPEAN PATENT SPECIFICATION

(11) **EP 1 196 623 B1**
(45) Date of publication and mention of the grant of the patent: **25.07.2007**
(21) Application number: 00948138.3
(22) Date of filing: 24.07.2000
(51) Int. Cl.: C12Q 1/00

(54) **METHOD FOR DETECTING SINGLE NUCLEOTIDE POLYMORPHISMS**
Verfahren zur Erkennung von Einzel-Nukleotid-Polymorphismen
AMELIORATIONS RELATIVES A L'ANALYSE D'ADN

(30) Priority: 23.07.1999 GB 9917307; 14.04.2000 GB 0009187
(43) Date of publication of application: 17.04.2002
(73) Proprietor: Forensic Science Service Ltd, London EC3N 2AA (GB)
(72) Inventor: GILL, Peter, The Forensic Science Service, Birmingham B5 6QQ (GB); HUSSAIN, Javaid, The Forensic Science Service, Birmingham B5 6QQ (GB); LONG, Adam, The Forensic Science Service, Birmingham B5 6QQ (GB)
(74) Representative: Pawlyn, Anthony Neil
(86) International application number: PCT/GB2000/002795
(87) International publication number: WO 2001/007640

(56) References cited:
- WO-A-93/25563
- GB-A- 2 312 747
- US-A- 5 710 028
- OLD J M: "DETECTION OF MUTATIONS BY THE AMPLIFICATION REFRACTORY MUTATION SYSTEM (ARMS)" METHODS IN MOLECULAR BIOLOGY,US,HUMANA PRESS INC., CLIFTON, NJ, vol. 9, 1991, pages 77-84, XP000775640
- WANG D G ET AL: "Large-scale identification, mapping, and genotyping of single-nucleotide polymorphisms in the human genome" SCIENCE,US,AMERICAN ASSOCIATION FOR THE ADVANCEMENT OF SCIENCE,, vol. 280, 1998, pages 1077-1082, XP002089398 ISSN: 0036-8075
- HOOGENDOORN B ET AL: "GENOTYPING SINGLE NUCLEOTIDE POLYMORPHISMS BY PRIMER EXTENSION AND HIGH PERFORMANCE LIQUID CHROMATOGRAPHY" HUMAN GENETICS,BERLIN,DE, vol. 104, 1999, pages 89-93, XP000938094

### SEQUENCE LISTING as ANNEX

<110> The Secretary Of State For The Home Department
<120> Improvements in and relating to analysis of DNA
<130> P17786wo
<140> PCT/GB00/02795
   <141> 2000-07-24
<150> GB9917307.2
   <151> 1999-07-23
<150> GB0009187.6
   <151> 2000-04-14
<160> 42
<170> PatentIn Ver. 2.1
<210> 1
   <211> 25
   <212> DNA
   <213> Artificial Sequence
<220>
   <223> Description of Artificial Sequence: An artificial universal primer sequence designed to act as a molecular beacon and referred to at page 13 of the application.
<400> 1
   acgcgctctc ttcttctttt gcgcg 25
<210> 2
   <211> 20
   <212> DNA
   <213> Artificial Sequence
<220>
   <223> Description of Artificial Sequence: An artificial universal reporter primer forward sequence designed to optimally prime at 60 degrees C, page 29.
<400> 2
   cgacgtggtg gatgtgctan 20
<210> 3
   <211> 20
   <212> DNA
   <213> Artificial Sequence
<220>
   <223> Description of Artificial Sequence: An artificial universal primer reverse sequence designed to optimally prime at approximately 60 degrees C, page 29.
<400> 3
   tgacctggct gactcgactg 20
<210> 4
   <211> 20
   <212> DNA
   <213> Artificial Sequence
<220>
   <223> Description of Artificial Sequence: An artificial universal primer reverse sequence designed to optimally prime at 60 degrees C, page 30.
<400> 4
   tgccgtggct gacctgagac 20
<210> 5
   <211> 20
   <212> DNA
   <213> Homo sapiens
<400> 5
   gtattttcgt ctggggggta 20
<210> 6
   <211> 21
   <212> DNA
   <213> Homo sapiens
<400> 6
   gtctgtcttt gattcctgcc c 21
<210> 7
   <211> 20
   <212> DNA
   <213> Homo sapiens
<400> 7
   tttgattcct gcctcatccc 20
<210> 8
   <211> 20
   <212> DNA
   <213> Homo sapiens
<400> 8
   atattacagg cgaacatacc 20
<210> 9
   <211> 27
   <212> DNA
   <213> Homo sapiens
<400> 9
   gcttgtagga cataataata acaatta 27
<210> 10
   <211> 22
   <212> DNA
   <213> Homo sapiens
<400> 10
   cagagatgtg tttaagtgct gt 22
<210> 11
   <211> 19
   <212> DNA
   <213> Homo sapiens
<400> 11
   accagctttg ccagttccm 19
<210> 12
   <211> 16
   <212> DNA
   <213> Homo sapiens
<400> 12
   ttccgtgggt gtggck 16
<210> 13
   <211> 21
   <212> DNA
   <213> Homo sapiens
<400> 13
   ggcagagcga ctaaaagcaa a 21
<210> 14
   <211> 37
   <212> DNA
   <213> Artificial Sequence
<220>
   <223> Description of Artificial Sequence: A human Gc forward primer with an artificial universal primer tag to detect a SNP polymorphism at Gels/1f, page 47.
<400> 14
   cgacgtggtg gatgtgctag gttccgtggg tgtggcc 37
<210> 15
   <211> 41
   <212> DNA
   <213> Artificial Sequence
<220>
   <223> Description of Artificial Sequence: A Human Gc reverse primer with an artificial universal primer tag to detect a SNP polymorphism at Ge1s/1f, page 47.
<400> 15
   tgacgtggct gacctgagac ggcagagcga ctaaaagcaa a 41
<210> 16
   <211> 45
   <212> DNA
   <213> Artificial Sequence
<220>
   <223> Description of Artificial Sequence: An artificial universal molecular beacon primer sequence designed to detect universal primer 9G polymorphism, page 47.
<400> 16
   acgcgctctc ttcttctttt gcgcgcgacg tggtggatgt gctag 45
<210> 17
   <211> 20
   <212> DNA
   <213> Artificial Sequence
<220>
   <223> Description of Artificial Sequence: An artificial reverse primer sequence designed to detect universal reverse 11 primer sequence, page 47.
<400> 17
   tgacgtggct gacctgagac 20
<210> 18
   <211> 39
   <212> DNA
   <213> Artificial Sequence
<220>
   <223> Description of Artificial Sequence: A human Gc forward primer attached to an artificial universal primer tag to detect a SNP polymorphism at Ge1s/1f, page 48.
<400> 18
   cgacgtggtg gatgtgctag accagctttg ccagttccg 39
<210> 19
   <211> 39
   <212> DNA
   <213> Artificial Sequence
<220>
   <223> Description of Artificial Sequence: A human Gc forward primer attached to an artificial universal primer tag to detect a SNP polymorphism at Gc1s/1f, page 48.
<400> 19
   cgacgtggtg gatgtgcttc accagctttg ccagttcct 39
<210> 20
   <211> 37
   <212> DNA
   <213> Artificial Sequence
<220>
   <223> Description of Artificial Sequence: A human Gc forward primer attached to an artificial universal primer tag to detect a SNP polymorphism at Gels/1f, page 48.
<400> 20
   cgacgtggtg gatgtgctag gttccgtggg tgtggcc 37
<210> 21
   <211> 37
   <212> DNA
   <213> Artificial Sequence
<220>
   <223> Description of Artificial Sequence: A human Gc forward primer attached to an artificial universal primer tag to detect a SNP polymorphism at Gc1s/1f, page 48.
<400> 21
   cgacgtggtg gatgtgcttc gttccgtggg tgtggca 37
<210> 22
   <211> 41
   <212> DNA
   <213> Artificial Sequence
<220>
   <223> Description of Artificial Sequence: A human Gc reverse primer attached to an artificial universal primer tag to detect SNP polymorphisms at Gc1s/1f, page 48.
<400> 22
   tgacgtggct gacctgagac ggcagagcga ctaaaagcaa a 41
<210> 23
   <211> 45
   <212> DNA
   <213> Artificial Sequence
<220>
   <223> Description of Artificial Sequence: An artificial molecular beacon forward primer attached to a universal primer tag to detect universal primer 9G polymorphism.
<400> 23
   acgcgctctc ttcttctttt gcgcgcgacg tggtggatgt gctag 45
<210> 24
   <211> 45
   <212> DNA
   <213> Artificial Sequence
<220>
   <223> Description of Artificial Sequence: An artificial molecular beacon forward primer attached to a universal primer tag to detect universal primer 9C polymorphism.
<400> 24
   acgcgctctc ttcttctttt gcgcgcgacg tggtggatgt gcttc 45
<210> 25
   <211> 20
   <212> DNA
   <213> Artificial Sequence
<220>
   <223> Description of Artificial Sequence: An artificial reverse universal primer designed to detect universal 11 sequence, page 48.
<400> 25
   tgacgtggct gacctgagac 20
<210> 26
   <211> 41
   <212> DNA
   <213> Artificial Sequence
<220>
   <223> Description of Artificial Sequence: A Human Amelogenin sequence forward primer attached to an artificial universal sequence to detect Amelogenin x polym.
<400> 26
   cgacgtggtg gatgtgcttc tgagccaatg gtaaacctgc c 41
<210> 27
   <211> 41
   <212> DNA
   <213> Artificial Sequence
<220>
   <223> Description of Artificial Sequence: A Human Amelogenin sequence forward primer attached to an artificial universal sequence to detect Amelogenin Y polym.
<400> 27
   cgacgtggtg gatgtgctag tgagccaatg gtaaacctgc a 41
<210> 28
   <211> 46
   <212> DNA
   <213> Artificial Sequence
<220>
   <223> Description of Artificial Sequence: A Human Amelogenin sequence reverse primer attached to an artificial universal sequence to detect Amelogenin X/Y polymorphism; n designates inosine..
<400> 28
   tgacgtggct gacctgagac cataggaagn gtactggtga gaaaca 46
<210> 29
<211> 45
   <212> DNA
   <213> Artificial Sequence
<220>
   <223> Description of Artificial Sequence: An artificial molecular beacon forward primer attached to a universal primer tag to detect universal primer 9G polymorphism.
<400> 29
   acgcgctctc ttcttctttt gcgcgcgacg tggtggatgt gctag 45
<210> 30
   <211> 45
   <212> DNA
   <213> Artificial Sequence
<220>
   <223> Description of Artificial Sequence: An artificial molecular beacon forward primer attached to a universal primer tag to detect universal 9C polymorphism, page 49.
<400> 30
   acgcgctctc ttcttctttt gcgcgcgacg tggtggatgt gcttc 45
<210> 31
   <211> 20
   <212> DNA
   <213> Artificial Sequence
<220>
   <223> Description of Artificial Sequence: An artificial reverse universal primer designed to detect universal 11 sequence, page 48.
<400> 31
   tgacgtggct gacctgagac 20
<210> 32
   <211> 37
   <212> DNA
   <213> Artificial Sequence
<220>
   <223> Description of Artificial Sequence: An artificial forward universal primer attached to human Gc1s sequence, page 57.
<400> 32
   ctagctggtg gctgtgctag gttccgtggg tgtggcc 37
<210> 33
   <211> 41
   <212> DNA
   <213> Artificial Sequence
<220>
   <223> Description of Artificial Sequence: An artificial reverse universal primer attached to human Gc sequence to detect Gc1s/1f polymorphisms, page 57.
<400> 33
   ctagctggtg gctgtgctag ggcagagcga ctaaaagcaa a 41
<210> 34
   <211> 42
   <212> DNA
   <213> Artificial Sequence
<220>
   <223> Description of Artificial Sequence: A human alpha-1- antitrypsin forward sequence attached to an artificial universal primer to detect alpha-1.M1S polym.
<400> 34
   ctagctggtg gctgtgctag aggggaaact acagcacctg ga 42
<210> 35
   <211> 42
   <212> DNA
   <213> Artificial Sequence
<220>
   <223> Description of Artificial Sequence: A human alpha-1- antitrypsin foward sequence attached to an artificial universal primer to detect alpha-1.S polym, Fig 11.
<400> 35
   ctagcctggt gtgtggctag aggggaaact acagcacctg gt 42
<210> 36
   <211> 43
   <212> DNA
   <213> Artificial Sequence
<220>
   <223> Description of Artificial Sequence: A human alpha-1- antitrypsin reverse sequence attached to an artificial universal primer to detect alpha-1.M1S polym.
<400> 36
   ctagctgctg tggtggctag tggtgatgat atcgtgggtg agt 43
<210> 37
   <211> 27
   <212> DNA
   <213> Homo sapiens
<400> 37
   cctgaagcca cacccacgga actggca 27
<210> 38
   <211> 18
   <212> DNA
   <213> Homo sapiens
<400> 38
   agttccgtgg gtgtggcc 18
<210> 39
   <211> 27
   <212> DNA
   <213> Homo sapiens
<400> 39
   cctgaggcca cacccacgga actggca 27
<210> 40
   <211> 27
   <212> DNA
   <213> Homo sapiens
<400> 40
   cctgaggcca cacccaagga actggca 27
<210> 41
   <211> 20
   <212> DNA
   <213> Artificial Sequence
<220>
   <223> Description of Artificial Sequence: Self complimentary universal forward reporter primer artificial sequence, Figure 25c.
<400> 41
   ctagctggtg gctgtgctag 20
<210> 42
   <211> 20
   <212> DNA
   <213> Artificial Sequence
<220>
   <223> Description of Artificial Sequence: Self complimentary universal reverse reporter primer artificial sequence, Figure 25c.
<400> 42
   ctagctggtg gctgtgctag 20

## Description

This invention concerns improvements in and relating to analysis of DNA, particularly, but not exclusively to techniques using single nucleotide polymorphisms for investigative purposes.

In forensic investigations, and analysis for other purposes, it is known to make use of bi-allelic markers or single nucleotide polymorphisms (SNPs). SNPs represent single base locations where variations between the sequence for one being and another can occur. A SNP may for instance be the presence of G or C, or of A or T, in the sequence of an individual, with some of the individuals having one of the options and other individuals having the other option. By considering a large number of such SNPs at different loci, a set of SNP results for an individual can be obtained which is useful for investigative purposes. The results may be compared with the results from another sample, with the statistical occurrence of that set of results within the population as a whole or used in other ways.

As each SNPs can only vary in being one of two options, a substantial number of different locations, generally several hundred loci, need to be investigated to achieve a set of results which is statistically significant in comparisons or other uses. Analysing such a large number of loci to determine the identity of SNP's on them is highly time consuming if the loci are considered individually, and introduces significant compatibility and reliability problems if multiplexes are used to analyse a number of those loci simultaneously.

GB-A-2312747 provides an SNP investigating technique. In a later amplification stage, however, it uses a single primer to amplify all products from the earlier amplifications. As a result an entirely separate and additional stage is need to investigate the SNP identities. WO-A-9325563 amplifies the sequence containing the target and then specifically probes for the target using probes which have a single tail sequence. Amplification using this tail sequence again means that the amplification process cannot distinguish in itself between identities: Methods in molecular Biology, US, Humana press Inc, Clifton, N.J. (1991) Vol 9, pp 77-84, XP775640 and Science; Vol 280. 15.05.1998, pp1077-1081, XP2089389 both provides a technique for SNP investigation but do not address the problems solved by the present invention.

The present invention has amongst other aims a technique for improving SNP based investigations, particularly where the original sample levels are very small. Improvements in the specifity of the results and in terms of the ease with which a very large number of such SNPs can be investigated simultaneously are also considered.

According to a first aspect of the invention we provide a method of investigating single nucleotide polymorphisms in a sample of DNA, the method comprising
contacting the DNA containing sample with a first set of primers, the first sets of primers including two or more primers including a locus specific portion and a further portion, the further portion of at least one of the primers being different from the further portion of at least one of the other primers,
amplifying the DNA, the amplification using the first sets of primers to give an amplified product, the amplified product including a sequence complementary to the locus specific portion and further portion of a first set primer;
contacting at least a portion of the amplified product with at least one second set of primers,
amplifying the first amplified product to give a further amplified product by hybridising at least one of the second set of primers to that part of the first amplified product with a sequence complementary to the further portion and
examining one or more characteristics of the further amplified product using the presence or absence of a distinctive unit introduced by hybridisation of a component, which includes the distinctive unit, to the sequence complimentary to the further portion.

According to a second aspect of the invention we provide a plurality of primers for investigating single nucleotide polymorphisms in the sample of DNA, the plurality of primers comprising two or more primers of a first set of primers and / or two or more primers of a second set of primers,
the first set of primers including two or more primers including a locus specific portion and a further portion, the further portion of at least one of the primers being different from the further portion of at least one of the other primers,
the second set of primers including at least one primer which has an equivalent sequence to the further portion of at least one of the first set of primers and hence will hybridise to a sequence complementary to the locus specific portion and further portion of a primer of the first set.

The first and / or second aspects of the invention may include any of the features, options or possibilities set out elsewhere in this application.

In one embodiment of the invention one or more, preferably all, of the first sets of primers may include two forward primers and a reverse primer. One or more, preferably all, of the first sets of primers may consist of two forward and a reverse primer. The forward primers and reverse primer preferably include sequences which anneal to the 3' and 5' sides respectively of the SNP at the locus incorporating the SNP under investigation.

In an alternative embodiment of the invention, one or more, preferably all of the first sets of primers may include a forward primer and a reverse primer. One or more, preferably all of the first sets of primers may consist of one forward primer and one reverse primer. The forward primer and reverse primer preferably include sequences which pair / anneal to the 3' and 5' sides respectively of the SNP at the locus incorporating the SNP under investigation.

The first set of primers may include one or more primers including a locus specific portion and a further portion. Preferably the forward primers are so provided. Preferably the further portion is attached to the 5' end of the locus specific portion, particularly in the case of forward primers. The 3' end of the forward primer is preferably provided with a SNP identifying portion. The further portion is preferably attached to the locus specific portion by a SNP related portion.

In one embodiment of the invention the locus specific portion preferably includes a sequence which matches the sequence of the locus sequence in the vicinity of the SNP under investigation. The match may occur at between 2 to 10 bases to the respective sides of the SNP under investigation. More preferably the sequence matches the locus sequence for the locus sequence adjacent to the SNP under investigation, ideally up to and including the nucleotide before the SNP on the 3' side of the SNP. Preferably the forward primers of a first set of primers are provided with identical sequences for the locus specific portion.

In one embodiment of the invention the SNP identifying portion is preferably a single nucleotide. The SNP identifying portion may be a C for investigating an SNP where the SNP may be a G nucleotide. The SNP identifying portion may be a G nucleotide for investigating an SNP where the SNP may be a C nucleotide. The SNP identifying portion may be a T nucleotide for investigating an SNP where the SNP may be an A nucleotide. The SNP identifying portion may be an A nucleotide for investigating an SNP where the SNP may be a T nucleotide. Preferably the SNP identifying portion for one forward primer of a set is one of C or G or A or T, with the SNP identifying portion of the other forward primer of the set being one of C or G or A or T, but different from the SNP identifying portion of the first forward primer of the set. Preferably the SNP identifying portions are provided to target the two possible variations of the SNP in question, for instance C and T for the primers to investigate G or A for the SNP, C or G for the primers to investigate G or C for the SNP and so on.

Preferably the SNP identifying portion forms the 3' end of the forward primers of the first set.

An exonuclease digestion prevention unit may be provided towards the 3' end of the forward primers. The exonuclease digestion prevention unit may be phosphorothioate. The exonuclease digestion prevention unit may be provided at the junction of the locus specific portion and SNP identifying portion.

The further portion preferably includes a sequence which does not match the locus sequence on the locus's 3' side of the locus sequence matching the locus specific portion of the primer. More preferably the sequence does not match the sequence of the locus in the vicinity of the SNP under investigation. Ideally the sequence does not anneal to, and particularly does not match, the sequence of any published part, ideally any part, of the entire DNA sequence of the entity from which the DNA containing the SNP under investigation was obtained, for instance Homo Sapiens. The inability of the sequence of the further portion to amplify human DNA is a particularly preferred feature. Preferably the forward primers of a first set of primers are provided with identical sequences for the further portion.

Preferably the further portion forms the 5' end of the forward primers of the first set.

The further portion of two or more of the forward primers of the first set may have an equivalent sequence. All the forward primers of the first set may be provided with further portions of equivalent sequence.

In a preferred embodiment of the invention, the further portion of at least one of the forward primers of the first set is different from the further portion of at least one of the other forward primers of the first set, at least in part. Preferably the further portion of each forward primer of the first set is different from the further portion of each of the other forward primers of the first set, at least in part. It is preferred that the forward primers are different from one another with respect to at least 25% of the nucleotides forming the further portion of the forward primers. Differences in sequence, ranging between 25% and 100% of the nucleotides forming the further portion of the forward primers may be employed. The differences in sequence may form one or more distinguishing portions. One or more distinguishing portions may be provided as or within the further portion of the forward primers. A distinguishing portion may be provided at the 5' end of the further portion of the forward primer. The distinguishing portion may be provided at the 3' end of the further portion of the forward primer. Preferably the distinguishing portion is provided at an intermediate location within the sequence of the further portion. Preferably a 5' end portion, distinguishing portion and 3' end portion defines the further portion of the forward primers.

The further portion of one or more of the primers in the first set may be provided with one or more portions which correspond with one or more portions in the further portion of one or more of the other primers in the first set. The nucleotides of the further portion of one or more of the forward primers may be equivalent to the nucleotides of one of the other forward primers, outside the distinguishing portion of the further portion. In particular, the 5' end portion and / or 3' portion of the further portion of one or more of the forward primers may be equivalent to the corresponding further portion of one or more of the other forward primers. Preferably all of the forward primers are provided with equivalent 5' end and/or 3' end portions to one another. The equivalent portions may form between 1 and 25% of the sequence of the further portion of the primers. Preferably the equivalent portions form between 10 and 25% of the sequence of the further portions. The reverse primer or primers of the first set may be provided with equivalent portions too.

The SNP related portion is preferably a single nucleotide. The SNP related portion is preferably identical to the SNP identifying portion of that primer. Preferably the two forward primers are provided with SNP related portions which are identical with their respective SNP identifying portions. The SNP related portion may be a C for investigating an SNP where the SNP may be a G nucleotide. The SNP related portion may be a G nucleotide for investigating an SNP where the SNP may be a C nucleotide. The SNP related portion may be a T nucleotide for investigating an SNP where the SNP may be an A nucleotide. The SNP related portion may be an A nucleotide for investigating an SNP where the SNP may be a T nucleotide. Preferably the SNP related portion for one forward primer of a set may be one of C or G or A or T, with the SNP related portion of another primer of the set being one of C or G or A or T, but different to the SNP related portion of the first primer of the set. Preferably the SNP related portions for the primers of a set are provided to match the SNP identifying portion of their respective primers.

Preferably during amplification the SNP related portion results in the amplified copies of the locus incorporating the SNP having an SNP repeat introduced into them. Ideally, the repeat has a base identity identical to that of the SNP.

Preferably the locus specific portion and SNP identifying portion of one of the forward primers anneals to the 3' side of the locus having the SNP under investigation. Preferably the locus specific portion and SNP identifying portion of another, ideally the other, of the forward primers does not anneal to the 3' side of the SNP under investigation. Preferably the annealing primer anneals due to a match between the SNP identifying portion and the SNP site, (for instance C matching to G). Preferably the non-annealing primer does not anneal due to a mis-match between the SNP identifying portion and the SNP site, (for instance, T mis-matching with T).

The SNP under investigation may be a location with variation between individuals of any two bases selected from C or G or A or T nucleotides. For instance, the SNP under investigation may be a location with variation between individuals of either a T or A nucleotide, T or C nucleotide, T or G nucleotide, A or C nucleotide, A or G nucleotide or C or G nucleotide. One possible variation may be investigated at one or more sites, with one or more other potential variations being investigated at one or more other sites.

Two or more SNP's may be investigated using a simultaneous first amplification and/or simultaneous second amplification and/or simultaneous examination of the one or more characteristic of the further amplified product. Preferably at least the first amplification and second amplification are conducted simultaneously for a plurality of SNP investigations. The number of SNP's investigated simultaneously in one or more stages of the process may be greater than 20, preferably greater than 25, more preferably greater than 50 and ideally greater than 100.

The sample may be a sample of DNA extracted from a collected source.

The sample may be contacted with the first primer set by mixing the sample and primers together.

The sample may be a mixture. One or more contributions to the sample may be analysed as the sample itself using the present invention. The mixed sample may include male and female DNA. One of the sexes of DNA, particularly the male, may be present in low concentrations relative to the other sex. For instance, the minor sex DNA contribution may form less than 1% of the sample, potentially less than 0.1 % and even less than 0.05%. The sample may contain samples from two or more sources. The method may investigate the minor sample in a mixture from two or more sources. The minor sample may form less than 1% of the mixed sample, potentially less than 0.1 % of the mixed sample and even less than 0.05% of the mixed sample.

The investigation may indicate the amount of DNA in a mixed sample from one or more of the sources. The indication may be based on a comparison of the experimentally determined results, for instance the level of a distinctive unit present, compared with a set of calibration results based on investigation of known amounts of DNA in a sample.

The first amplification is preferably performed by PCR. The amplification preferably involves between 18 to 60 cycles, more preferably 20 to 40 cycles.

The amplification cycles, particularly where the first and second amplification processes are used, may have the following characteristics. Preferably the amplification cycles include a first cycle set in which the annealing temperature of the cycle is similar or above the melting temperature of the first set of primers, particularly of the locus specific portion of the first set of primers and / or similar or above the second set of primer. The amplification cycles may include a second set of cycles, with preferably, the annealing temperature in the second set of cycles being similar or below the melting temperature of the first set of primers and / or above the melting temperature of the second set of primers. The melting temperature of the first set of primers may rise after one or two cycles. The amplification cycles may include a third set of cycles, with, preferably, the annealing temperature in the third set of cycles being below the melting temperature of the first set of primers and / or similar or above the melting temperature of the second set of primers.

It is preferred that the first set of cycles provide between 2 and 10 cycles. It is preferred that the second set of cycles provide between 3 and 15 cycles. It is preferred that the third set of cycles provide between 15 and 35 cycles. Preferably the total of cycles provided in the first, second and third sets does not exceed 40 cycles.

It is preferred that the denaturation temperature for the first and / or second and / or third set of cycles be 92 to 96°C, ideally 94°C.

It is preferred that the annealing temperature for the first and / or second and / or third set of cycles be between 60 and 62°C, ideally 61 °C. It is preferred that the annealing temperature for the second set of cycles be between 70 and 78°C, ideally between 72 and 75°C.

It is preferred that the extension temperature for the first and / or second and / or third set of cycles be between 70 and 75°C, ideally 72°C.

Amplification preferably results in extension of the annealed forward primer from its 3' end towards the 5' end of the target sequence. Amplification preferably results in extension of the reverse primer from its 3' end towards the 5' end of its target sequence. Preferably further cycles of amplification result in extension of the forward primer sequence towards the 5' end of its target, including the reverse primer sequence. Preferably further cycles of amplification result in extension of the reverse primer sequence towards the 5' end of its target, including one or more or all of the forward primer sequence and particularly the SNP identifying portion, locus specific portion, SNP related portion and further portion.

A portion of the amplified product may be removed and contacted separately with the second set of primers. Contact with the second set of primers may occur in a separate vessel to the contact with the first set of primers. This is particularly preferred where universal primers incorporating molecular beacons are used. Preferably a two tube and / or branched PCR process is used where universal primers incorporating molecular beacons are employed.

The first and second amplifications may occur in the same vessel. The first and second amplifications may occur substantially simultaneously. Preferably the method includes adding one or more of the first set of primers and one or more of the second set of primers to the sample to be amplified prior to conducting amplification cycles.

The one or more first sets of primers may be provided at a concentration of between 20 and 80nM, more preferably between 40 and 60nM and ideally at 50nM +/- 5%. Preferably the primers which do not compete and/or for which site overlap does not occur are provided at these levels. Where primer competition could occur and/or where primer site overlap occurs preferably the primer's relative concentrations are balanced. The reverse primer concentration for such a simultaneous process may be between 75nM and 125nM, for instance 100nM +/- 10%.

The second set of primers may be provided at a concentration of between 20 and 80nM, more preferably between 40 and 60nM and ideally at 50nM +/- 5%. The amount of the second set of primers added may be defined by Cn x L, where Cn is the concentration of the primers and L is the number of loci under consideration +/- 2 and ideally is the number of loci under consideration, particularly where L is less than 100 or even less than 50. Preferably the maximum second set of primers concentration is 1000nM.

Particularly where the first and second sets of primers are present together, it is preferred to provide the second set of primers and first set of primers at a concentration ratio of at least 5:1. A ratio of at least 10:1, more preferably at least 20:1 and ideally at least 30:1, second set concentration: first set concentration may be provided. The first set may be provided at a concentration of between 5 and 400nM, more preferably between 10 and 200nM. The second set may be provided at a concentration of between 300nM and 5000nM, more preferably between 400 and 4000nM.

Particularly where the first and second sets of primers are present together, it is preferred to use an annealing temperature at which at least 80% of the second set of primers remain single stranded, more preferably a temperature at which at least 95% of the second set of primers remain single stranded and ideally a temperature at which at least 99% of the second set of primers remain single stranded, for some of the cycles of the amplification process. A lower annealing temperature may be used for other cycles of the amplification process. Preferably the higher temperature annealing is used at least in cycles 3 to 30, more preferably in cycles 3 to 40. A lower annealing temperature may be used in the first two cycles. A lower annealing temperature is preferably used in at least the last two cycles. The lower annealing temperature is preferably a temperature at which at least 80%, more preferably at least 90% and ideally at least 99% of the second set of primers anneal.

The amplified product may be contacted with the second primer set by mixing the sample and primers together.

The second set of primers may include one, two, three or four forward primers. A reverse primer may be present, but the second set of primers may lack a reverse primer.

The invention may only provide one second set of primers provided. In one embodiment of the invention preferably the one second set of primers consisting of two forward primers and a reverse primer. One or more, preferably all, of the second sets of primers may include two forward primers and a reverse primer. One of the forward primers of the second set preferably includes a sequence which anneals to the SNP incorporating strand on the 3' side of the SNP. The reverse primer of the second set preferably includes a sequence which anneals to the 3' side of the base pairing to the SNP. More preferably one of the forward primers includes a sequence which anneals to the 3' side of the SNP repeat. Preferably the other forward primer or primers does not anneal.

In the one embodiment of the invention the second set of primers may include one or more primers including a second further portion. Preferably the forward primers are so provided. Preferably the second further portion is provided with a second SNP identifying portion and / or more preferably an SNP repeat identifying portion. The second SNP or SNP repeat identifying portion may be attached to the 3' end of the second further portion, particularly in the case of forward primers. The 5' end of the forward primer is preferably provided with a distinctive unit.

In the one embodiment of the invention the second further portion preferably includes a sequence which pairs to the sequence of the amplified product in the vicinity of the SNP identifying portion and/or, more preferably, SNP repeat related portion thereof. More preferably the further portion sequence adjacent to the SNP related portion, ideally up to and including the nucleotide before the SNP related portion matches the sequence of the amplified product adjacent to the SNP repeat, ideally up to and including the nucleotide before the SNP repeat. Preferably the forward primers of a second set of primers are provided with identical sequences for the second further portions.

In an alternative embodiment of the invention it is preferred that the one second set of primers consists of one forward primer and one reverse primer. One or more, preferably all, of the second set of primers may consist of one forward primer and one reverse primer. Preferably the forward primer of the second set includes a sequence which anneals to the SNP incorporating strand on the 3' side of the SNP. Preferably the reverse primer of the second set includes a sequence which anneals to the 3' side of the base pairing to the SNP. Most preferably the forward primer includes a sequence which anneals to the sequence which pairs to the sequence produced by the copying of the further portion of the forward primer and / or which corresponds to the sequence of the further portion of the forward primer of the first set.

In the alternative embodiment of the invention, the second set of primers may include a primer including a second further portion and more preferably consisting of a second further portion. Preferably the forward primer is so provided. Preferably the second further portion is provided with a sequence which pairs to the sequence of the amplified product in the vicinity of the sequence which pairs to the further portion of the forward primer of the first set. More preferably, the second further portion includes a sequence which matches the sequence of the first further portion and / or pairs to the sequence of the amplified product matching the first further portion.

Preferably the sequence of the second further portion does not anneal to, and particularly does not match, the sequence of any published part, ideally any part, of the entire DNA sequence of the entity from which the DNA containing the SNP under investigation was obtained, for instance Homo Sapiens. The inability of the sequence of the second further portion to amplify human DNA is a particularly preferred feature. Preferably the forward primers of a second set of primers are provided with identical sequences for the second further portion.

In the one embodiment of the invention the second SNP related portion is preferably a single nucleotide or two nucleotides.

In the one embodiment of the invention preferably the second SNP related portion of one primer of the second set is, or includes, a nucleotide which is identical to the SNP identifying portion and/or SNP related portion of a primer of the first set. Preferably another, ideally the other, primer of the second set has a second SNP related portion which is, or includes, a nucleotide which is identical to the SNP identifying portion and/or SNP related portion of another, ideally the other, primer of the first set.

In the one embodiment of the invention where a single nucleotide forms the second SNP related portion, the second SNP related portion may be a C nucleotide when amplifying a target in which the SNP or SNP repeat is a G nucleotide. The second SNP related portion may be a G nucleotide when amplifying a target in which the SNP or SNP repeat is a C nucleotide. The second SNP related portion may be a T nucleotide when amplifying a target in which the SNP or SNP repeat is an A nucleotide. The second SNP related portion may be an A nucleotide when amplifying a target in which the SNP or SNP repeat is a T nucleotide. The second SNP related portion for one forward primer of a second set may be one of C or G or T or A with the second SNP related portion of another primer of the second set being one of C or G or A or T, but different to the second SNP related portion of the first primer of that set where the SNP or SNP repeat under investigation could be any two of C or G or T or A nucleotides.

In the one embodiment of the invention the second SNP related portion may be formed of two nucleotides. Preferably the end nucleotide of the two matches with the nucleotide of the SNP or SNP repeat of interest. Preferably the nucleotide adjacent to the end nucleotide of the second SNP related portion is a mismatch with the base adjacent to the SNP or SNP repeat in the target sequence.

In the one embodiment of the invention preferably the second SNP related portion forms the 3' end of the forward primers of the second set.

An exonuclease digestion prevention unit may be provided towards the 3' end of the forward primer or primers of the first and / or second set. The exonuclease digestion prevention unit may be phosphorothioate. The exonuclease digestion prevention unit may be provided at the end of the second further portion and / or the junction of the second further portion and second SNP related portion.

Preferably the second further portion and / or second SNP related portion of the forward primer and / or of one of the forward primers anneals to the 3' side of the SNP or SNP repeat. Preferably the second further portion and / or second SNP related portion of another, ideally the other, of the forward primer and / or of the forward primers does not anneal to the 3' side of the SNP and / or SNP repeat. In one embodiment of the invention preferably the annealing primer anneals due to a match between the second SNP related portion and the SNP repeat and / or adjacent sequences. Preferably the non-annealing primer does not anneal due to a mis-match between the second SNP related portion and the SNP repeat. In an alternative embodiment of the invention preferably the annealing primer anneals due to a match between the second further portion and a sequence which paired to the first further portion.

The second amplification is preferably performed by PCR. The amplification preferably involves between 18 and 30 cycles, more preferably 20 to 25 cycles.

One or more of the primers of the first and/or second set may be provided with one or more portions which are complimentary to one or more portions on one or more of the other primers in that set. The complimentary portion or portions are preferably provided in the further portion of the primers of the first set. The complimentary portion or portions are preferably provided in the second further portion of the primers of the second set. Preferably a complimentary portion is provided on each of the primers of a set. Preferably at least two complimentary portions are provided on each of the primers of a set. Preferably a complimentary portion is provided at the 3' end of a primer, ideally all the primers. Preferably a complimentary portion is provided at the 5' end of a primer, ideally all of the primers. Preferably the 3' end complimentary portion of one primer is complimentary to the 5' end complimentary portion of another primer, ideally all the other primers of the set and/or both sets. Preferably the 5' end complimentary portion of one primer is complimentary to the 3' end complimentary portion of another primer, ideally all the other primers of the set and/or both sets. A locus specific portion may be provided on the further portion including the complimentary portion or portions, particularly on the 3' end. The further portion and/or second further portion may include a sequence matching the sequence of the locus under consideration, particularly provided between two complimentary portions. The complimentary portions may be at least 3 nucleotides long, more preferably between 3 and 20 nucleotides long. The complimentary portions are preferably both of the same length. The complimentary portions may form between 5 and 40% of the further portion and/or second further portion. One, two, three or four primers of a set may be provided in this way. Preferably the reverse primer or primers are similarly provided.

The further amplified product, or a portion thereof, may be removed from the vessel in which the amplification is performed to examine the one or more characteristics. Alternatively or additionally, the one or more characteristics may be examined with the further amplified product in the vessel in which amplification is performed.

The one or more characteristic of the further amplified product may be examined by means of the presence and/or absence of a distinctive unit in the further amplified product. The distinctive unit may be incorporated in the further amplified product or be associated there with. The distinctive unit may be introduced during the amplification process and / or in a subsequent step. The subsequent step may comprise hybridisation, for instance, of a component to the SNP base. The component may be a dideoxynucleotide, particularly a dideoxynucleotide incorporating a distinctive unit such as a dye.

The distinctive unit may be a dye label or colour producing molecule.

The distinctive unit may be a sequence of DNA, for instance a molecular beacon. The sequence of DNA, for instance a molecular beacon, may comprise a sequence of DNA incorporating a dye molecule. The sequence of DNA may be a single strand. The sequence of DNA may be looped by joining one part of the sequence to another. Preferably the dye molecule is in the loop, still more preferably in one part of the sequence which is joined to another. Preferably the dye molecule is in proximity with a quencher molecule. Preferably the quencher molecule prevents the dye molecule characteristic, for instance fluorescence, being visible. Preferably the dye molecule becomes visible, for instance fluorescent, upon activation. Preferably activation is caused by primer extension into the sequence of the molecular beacon. Activation preferably occurs through the opening of the loop. The molecular beacon sequence may be F-ACGCGCTCTCTTCTTCTTTTGCGCG-Q where F is a distinctive unit such as a dye, and Q is a quenching unit or vice versa. Preferably the parts of the molecular beacon sequence which join to one another are the stems ACGCGC from the 5' end and GCGCG from the 3' end. Preferably the universal primer incorporating molecular beacon does not contain phosphorothioate bonding. Preferably none of the second set of primers contain phosphorothioate bonding. Ideally none of the first or second primers contain phosphorothioate bonding. Where molecular beacons are used, the amplification product may be examined for one or more characteristics in the amplification reaction vessel. For instance, the Roche Light Cycler™ or other such instruments may be used for this purpose.

The distinctive unit may be visible under daylight or conventional lighting and/or may be fluorescent.

The distinctive unit may be an emitter of radiation, such as a characteristic isotope.

The distinctive unit is preferably provided at the 5' end of one or the primers, more preferably on a forward primer and ideally with a different distinctive unit for the other forward primer of the second set.

Preferably the distinctive unit is indicative of the nucleotide present at the SNP. Preferably a different distinctive unit is present if one nucleotide is present at the SNP and than if the other nucleotide is present at the SNP. Different distinctive units may be provided for indicating the SNP at one locus when compared with the distinctive units for indicating the SNP present at a different locus.

The examination may involve separating the further amplified product relating to one SNP from the further amplified product from one or more other SNP's. Preferably the further amplified products for each SNP are separated from one another. Electrophoresis may be used to separate one or more of the further amplified products from one another. The further amplified products may be separated from one another based on size of the further amplified products, for instance due to the different length of the further amplified products.

The examination may involve analysing the response of the further amplified product, for instance in the vessel in which amplification was performed, to radiation of various wavelengths, for instance fluorescent light.

The examination may involve the use of micro-fabricated arrays. The further amplified product may be contacted with one or more components retained on a solid support. One or more of the components may be an oligonucleotide, preferably with its 5' end tethered to the support. Preferably the oligonucleotide has a sequence which pairs / anneals with the sequence of at least one, ideally only one, of the further amplified products.

In an embodiment the oligonucleotide may have a sequence which pairs / anneals with the sequence of at least one, ideally only one, of the further amplified products up to the base before the base which is the SNP site. Only a portion of the further amplified product may pair / anneal to the oligonucleotide. Preferably a particular further amplified product type pairs / anneals to a particular oligonucleotide.

In an another embodiment, the oligonucleotide may have a sequence which pairs / anneals with the sequence of at least one, ideally only one, of the further amplified products along the sequence corresponding to the locus specific portion and the further portion. Preferably the further portion of the further amplified product includes a distinctive unit. The distinctive unit is preferably a dye. Preferably a different dye is present on each different further amplified product.

A plurality of such components, such as a plurality of oligonucleotides may be provided. A plurality of different oligonucleotides may be provided with each having a sequence which pairs / anneals to a further amplified product, ideally only one such product. It is particularly preferred that each oligonucleotide type pairs / anneals to a different further amplified product type from the others. The plurality of different types of oligonucleotides may be provided at a plurality of different, ideally discrete locations on the support.

The solid support may be glass, silicon, plastics, magnetic beads or other materials.

In an embodiment one form of the invention, the oligonucleotide and paired / annealed further amplified product may be contacted with one or more further components. Preferably one or more of the further components includes a dideoxynucleotide. Preferably one or more of the further components includes a distinctive unit, such as a dye. Preferably different further component types include different distinctive units. Two or more components comprising two or more different dideoxynucleotides with a different distinctive unit attached to each may be provided. The dideoxynucleotides may be A, T, C or G. Three or four dideoxynucleotides may be provided, preferably each with a different distinctive unit.

One or more, preferably only one of the further components may selectively attach to the SNP base and / or 3' end of the oligonucleotide. Preferably the selectivity of the attachment is based on the pairing of part of the further component's identity with the SNP base identity, such as the pairing of the dideoxynucleotide identity with the SNP base identity. Preferably the pairing incorporates the distinctive unit in the structure. Preferably the pairing incorporates the distinctive unit in the structure. Preferably non-pairing further components and their distinctive units are not incorporated in the structure.

The identity of the distinctive unit attached to the component in the structure is preferably investigated. Preferably the identity of the further component and / or the identity of the SNP is derived from the identity of the distinctive unit.

In another form of the invention, the oligonucleotide and paired / annealed further amplified product may be contacted with one or more additional components. The one or more additional components may be one or more further oligonucleotides. Preferably one or more of the additional components includes an end base, preferably at its 5' end. Preferably one or more of the additional components includes a distinctive unit, such as a dye. Preferably different additional component types include different distinctive units. The additional components may comprise two or more different further oligonucleotides with a different distinctive unit and / or end base attached to each. The end base of the further oligonucleotides may be C, G, A or T. Three of four further oligonucleotides may be provided, preferably each having a different distinct unit and / or end base.

One or more, preferably only one, of the further oligonucleotides may selectively attach to the SNP base and / or 3' end of the tethered oligonucleotide. Preferably the selectivity is based on the pairing of the further oligonucleotide's end base identity with the SNP base identity. Ligase may be provided in contact with the tethered oligonucleotide and / or further oligonucleotide and / or further amplified product. Preferably ligation occurs where the SNP base and end base pair, thereby incorporating the distinctive unit in the structure. Preferably non-pairing further components and the distinctive units are not incorporated in the structure.

The identity of the distinctive unit attached to the component in the structure is preferably investigated. Preferably the identity of the additional component and / or the identity of the end base of the additional component and / or the identity of the SNP is derived from the identity of the distinctive unit.

In yet another embodiment of the invention the further amplified product may incorporate an attachment unit. Preferably the attachment unit facilitates attachment of the further amplified product to a solid support. The solid support may be glass, silicone, plastics, magnetic beads or other materials. Preferably attachment is affected by means of a covalent bond. The attachment unit may be an amino group, preferably an amino group provided at the 5' end of the further amplified product. It is preferred that the solid support is an epoxy-silane treated support in such cases. The attachment unit may be a phosphorothiate unit, ideally provided at the 5' end of the further amplified product. In such a case, attachment to a bromo-acetomide treated solid support is preferred.

The further amplified product, attached to a solid support, is preferably contacted with one or more probes preferably having a different sequence from one another, at least in part. Preferably each probe has a common sequence portion to each other probe. It is particularly preferred that this common sequence portion correspond in sequence to the locus specific portion of the further amplified product. Preferably the probes incorporate at least one different sequence portion compared with one another. Preferably the different portions, for at least one of the probes, corresponds to the universal primer portion sequence of the further amplified product. It is preferred that contact of the probes with the further amplified product results in hybridisation of one of the probes to the further amplified product, ideally with no hybridisation of the other probe or probes. Preferably each probe has a distinctive unit attached, such as a dye unit. Preferably different distinctive units are used for each different probe.

The sample may be compared with another sample. The comparison may be based on comparing one or more of the one or more characteristic of the further amplified products for each sample. The samples may be compared to confirm a match in the characteristic between the samples. The samples may be compared to eliminate a match in the characteristic between the samples. The occurrence of the one or more further characteristic for one or more SNP's may be compared with information on the frequency of occurrence of the one or more further characteristic for the one or more SNP's in a population. The population may be a representative sample of the population of a country, an ethnic group or database.

According to another embodiment of the invention we provide a method of investigating a sample of DNA, the method comprising contacting the DNA containing sample with two or more primers, amplifying the DNA using those primers to give an amplified product and examining one or more characteristics of the amplified product, at least a first set of amplification conditions and a second set of amplification conditions being employed, amplification by one or more of the primers being at least impaired during the use of one or the sets of amplification conditions.

Preferably amplification by one or more of the primers is inhibited during the use of one at least one of the sets of amplification conditions. Preferably annealing of the one or more primers is inhibited during the use of at least one of the sets of amplification conditions. Preferably one or more of the primers remains single stranded during the annealing step of one or more of the sets of amplification conditions.

The first set of amplification conditions may inhibit amplification by one or more of the primers. The second set of amplification conditions may inhibit amplification by one or more of the primers. One or more further sets of amplification conditions may be provided. One or more of the one or more further sets of amplification conditions may impair and / or inhibit amplification by one or more of the primers.

In one embodiment, the first set of amplification conditions may impair or inhibit amplification by one or more of the primers. Preferably the second set of amplification conditions provides for amplification by the previously impaired or inhibited one or more primers, particularly by all primers.

In another embodiment, the first set of amplification conditions may not inhibit or impair amplification by the primers, though amplification by one or more of the primers may be impaired or inhibited by other factors, the second set of conditions may inhibit or impair amplification by one or more of the primers and a third set of conditions may be provided in which the one or more primers impaired or inhibited by the second set of conditions are no longer inhibited or impaired.

In yet another embodiment, one or more primers may be inhibited during the first set of amplification conditions, not inhibited during a second set of amplification conditions, and not inhibited during a third set of amplification conditions. Particularly in such a case one or more other primers may remain inhibited during the first and second set of amplification conditions, but not during the third set of amplification conditions.

A set of amplification conditions may include a denaturation stage, annealing stage and extension stage. Preferably a set of amplification conditions is applied for a number of cycles. The extension stage may be provided during the cycles of one or more sets of conditions for between 30 seconds and 3 minutes, preferably 2 minutes +/- 10%. The denaturation stage may be provided during the cycles of one or more sets of conditions for between 15 and 60 seconds, preferably 30 seconds +/- 10%. The annealing stage may be provided during the cycles of one or more sets of conditions for between 15 and 60 seconds, preferably for 30 seconds +/-10%. A temperature of between 70 and 80°C, more preferably between 74 and 78°C and ideally 76°C is preferably provided for the extension stage. A denaturation temperature of between 85 and 98°C, more preferably between 92 and 96°C and ideally 94°C is preferably provided.

An annealing temperature of less than 72°C is preferably provided in a set of amplification conditions allowing annealing of two or more primers and / or not inhibiting any primer annealing. Preferably a temperature of 72 °C or greater is employed in one or more sets of amplification conditions intended to impair or inhibit amplification by one or more of the primers.

The amplification process may be performed for between 1 and 20 cycles, particularly the first one to three cycles, where one or more of the primers is impaired or inhibited from annealing. Preferably between 10 and 80 cycles are employed where the primers are not impaired of inhibited. The overall process may include between 40 and 80 cycles, more preferably between 45 and 70 cycles, and ideally between 50 and 70 cycles.

The method may use one or more of the primers, preferably for all of the primers which are not inhibited or impaired during all the sets of conditions, at a concentration of between 5 and 500nM, more preferably at between 10 and 200nM.

Various embodiments of the invention will now be described, by way of example only, and with reference to the accompanying drawings in which:-
Figure la to le illustrates the various parts of the first stage of a process according to the present invention;
Figure 2a illustrates one forward primer suitable for use in the present invention;
Figure 2b illustrates a second forward primer suitable for use in the present invention and intended for use with the primer of Figure 2a;
Figure 3a to 3e illustrates the various parts of the second stage of a process according to the present invention;
Figure 4a illustrates a "universal" forward primer suitable for use in the second stage of the present invention;
Figure 4b illustrates a second "universal" forward primer for use in the second stage of the present invention and intended for use with the primer of Figure 4a;
Figure 5 illustrates schematically the products of the two stage process when applied in a multiplex system;
Figure 6 illustrates amplification in the first stage using a primer according to the present invention;
Figure 7a schematically illustrates a structure for providing an examinable characteristic for the further products of the present invention;
Figure 7b illustrates the sequence of the structure of Figure 7a;
Figure 7c illustrates an "universal" forward primer incorporating a molecular beacon having one universal primer sequence;
Figure 7d illustrates a further "universal" forward primer incorporating a molecular beacon having a different primer sequence;
Figures 8a to 8f illustrate an alternative amplification process according to the present invention;
Figures 9a to 9d illustrate a micro-fabricated array investigation technique for amplifying products according to the present invention, based on genetic bit analysis;
Figures 10a to 10e illustrate a micro-fabricated array investigation for the amplified products of the present invention based on a ligation technique;
Figure 11 illustrates two forward primers and a reverse primer suitable for use in one embodiment of the first stage amplification process of the present invention;
Figures 12a to 12e illustrate various features of a hybridisation based investigation of the amplification result where the amplified strand is tethered to a glass slide;
Figures 13a and 13b illustrate a further way of investigating the results by hybridising the amplified products with oligonucleotides tethered to glass slides;
Figure 14 illustrates results for three samples and a control, at four loci, using the present invention;
Figure 15 illustrates the sensitivity and specifity of the technique of the present invention;
Figure 16a primer 416a tested against Gc 1 S 1 S; 1 F-1 F; 2-2 and negative control, illustrating that only 1 F-1 F gives a signal and no background was observed with the other samples;
Figure 16b illustrates a simplex reaction to test specifity of the forward primer 420G, which detects both Gc1 polymorphisms, the primer being tested against a series of individuals, Gc1S-1S; 1F-1F; 2-2 and a negative control, with only the first two samples giving a signal with the remainder being clear;
Figure 16c illustrates a simplex reaction to test specifity of the forward primer 420T which detects Gc2, with samples taken from 1S-1S, 1F-1F, 2-2 and negative control, again with no background being detected;
Figure 16d demonstrates the low levels of sample which can be detected, the test being conducted on a series of samples taken from individual 1S-1S and prepared at 1NG, 200PG, 400PG and 8PG respectively, (Figures top to bottom in order), with a cocktail of GC420; GC416 primers and the universal primers used in the PCR reaction, the results indicating, as both bits are green, that priming by 416C and 420G thoroughly occurred, this being consistent with the known genotype of the individual, with consequently no background from 416A or 420T being observed;
Figure 17is a graph of fluorescence verses cycle number for samples amplified with Gc1s primer and varying concentrations of second round universal primer;
Figure 18 is a minigel result for PCR products according to an amplification performed according to the invention;
Figure 19 is a graph of fluorescence verses cycle number for reduced concentrations of Gc1s + ve DNA compared to a control, SDW;
Figure 20a is a graph of fluorescence against amplification cycle number for Gc1 primer with universal G beacon for DNA samples 1, 2 and control sample 3;
Figure 20b is a graph of fluorescence against amplification cycle number for Gc2 primer with universal C beacon for the samples of Figure 17a;
Figure 20c is a graph of fluorescence against amplification cycle number for Gc1s primer with universal G beacon for the samples of Figure 17a;
Figure 20d is a graph of fluorescence against amplification cycle number for Gc1f primer with universal C beacon for the samples of Figure 17a;

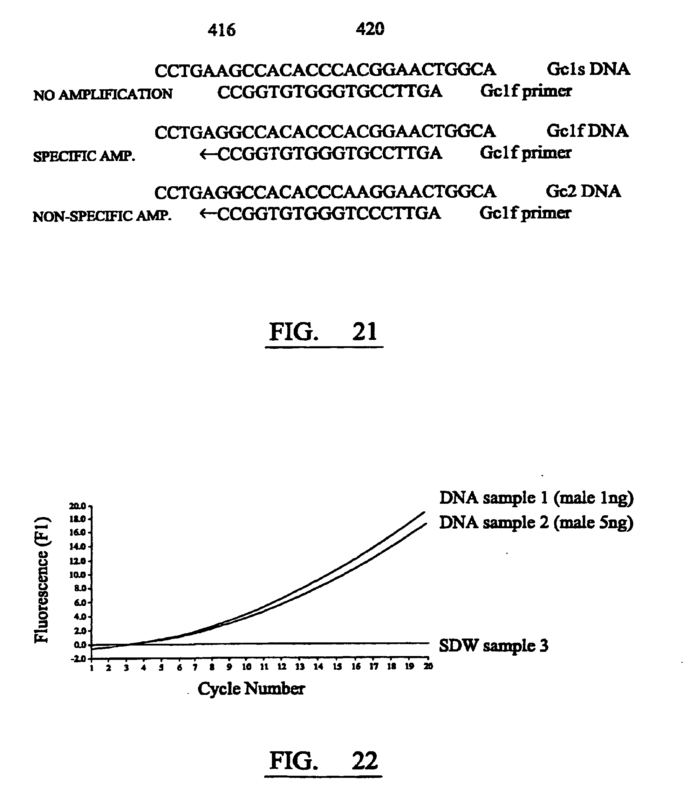

Figure 21 is an illustration of the primer binding sites of Gc I f primer in respect of codon positions 416 and 420 on samples that are Gc1s, Gc1f and Gc2 phenotypes;
Figure 22 is a graph of fluorescence against amplification cycle number for male DNA sample with amelo Y primer and universal G beacon;
Figure 23a is a graph of fluorescence against amplification cycle number for the major component of a 2 DNA sample mixture coding for the Gc2 polymorphism and varying major to minor component ratios;

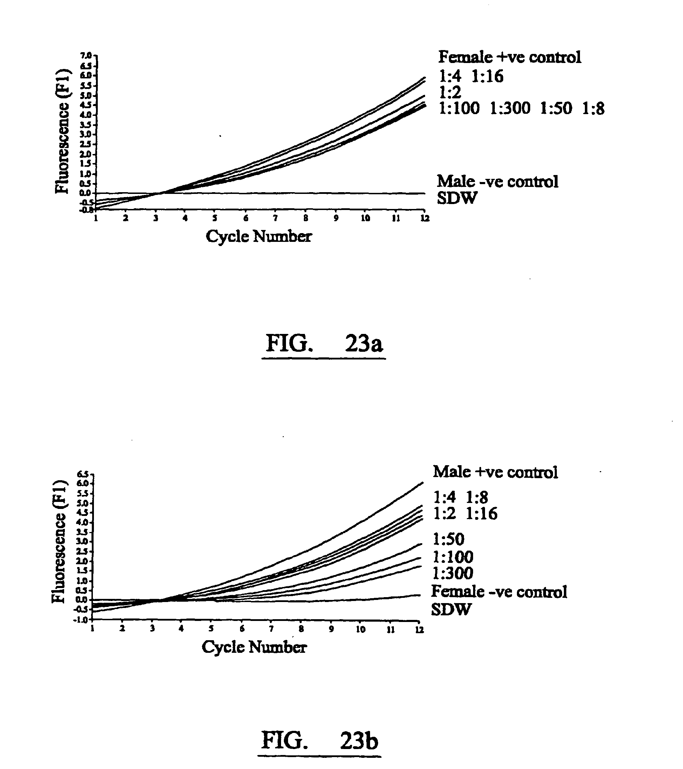

Figure 23b is a graph of fluorescence against cycle number showing the detection of the minor component of a 2 sample DNA mixture coding for the Gc1s polymorphism with varying major to minor concentration ratios;
Figure 24 is an illustration of experimental results with amplification at different annealing temperatures illustrating extensive primer dimer formation at 70°C and 72°C, diminished by the primer dimer formation at 74°C and substantially no primer dimer formation at 76°C;
Figure 25a illustrates primers susceptible to primer dimer formation;
Figure 25b illustrates two primers for which a primer dimer formation cannot occur;
Figure 25c illustrates primer sequences according to the concept of Figure 25b;
Figure 26 is a graph of fluorescence verses cycle numbers for different samples containing different amounts of DNA, a DNA negative sample and two sterile distilled water samples;
Figure 27 is a graph of fluorescence against cycle number for samples amplified using a particular primer set, again illustrating samples containing various levels of DNA, a DNA negative sample and two sterile distilled water samples; and
Figure 28 illustrates a graph of fluorescence verses cycle number for samples amplified using a particular primer set, various samples containing varying levels of DNA being provided, together with a DNA negative sample and two samples of sterile distilled water.

The nucleotide sequence of humans and other biological entities is in a large part consistent between individuals. Locations are known, however, at which variation occurs. One such form of variation is known as single nucleotide polymorphisms or bi-allelic markers, where the identity of a single nucleotide at a specific location is one of four possibilities from any of the four bases available, A, T, G or C. In many cases the variation is only bi-allelic and hence only one or two possibilities applies. Thus, some individuals may have a sequence incorporating a C base at a particular position, whereas other individuals will have a G base at that position; the surrounding sequences for both individuals being identical.

Medical diagnostics, forensic investigations and other DNA tracing applications make use of such single nucleotide polymorphisms (SNPs) for identification purposes. As the variation between individuals can only be between one of two options, a very substantial number of such locations, loci, must be considered for a statistically significant result, for instance the statistical significance of a match between a collected sample and an individual's makeup to be obtained.

Investigating such a large number of loci, frequently several hundred, on an individual basis is extremely time consuming. To reduce the time taken, it might be desirable to construct multiplexes which allow a substantial number of loci to be investigated simultaneously based on PCR or other amplifying techniques. The design of reliable constructs for a large number of loci, however, is extremely difficult due to problems in interactions between the primers needed for the different loci, different conditions for suitably efficiency amplification of the different primers and a variety of other issues.

The technique of the present invention is designed to simplify SNP based and other investigations, and particularly to facilitate the rapid development of multiplexes suitable for investigating a large number of such loci simultaneously, due to the flexibility offered by the present technique.

The technique is based around two amplification stages, generally achieved through PCR, with both of the stages offering specifity in terms of the SNPs identified and amplified. The two amplification stages can be conducted separately or simultaneously and the amplification products can be analysed in a variety of ways.

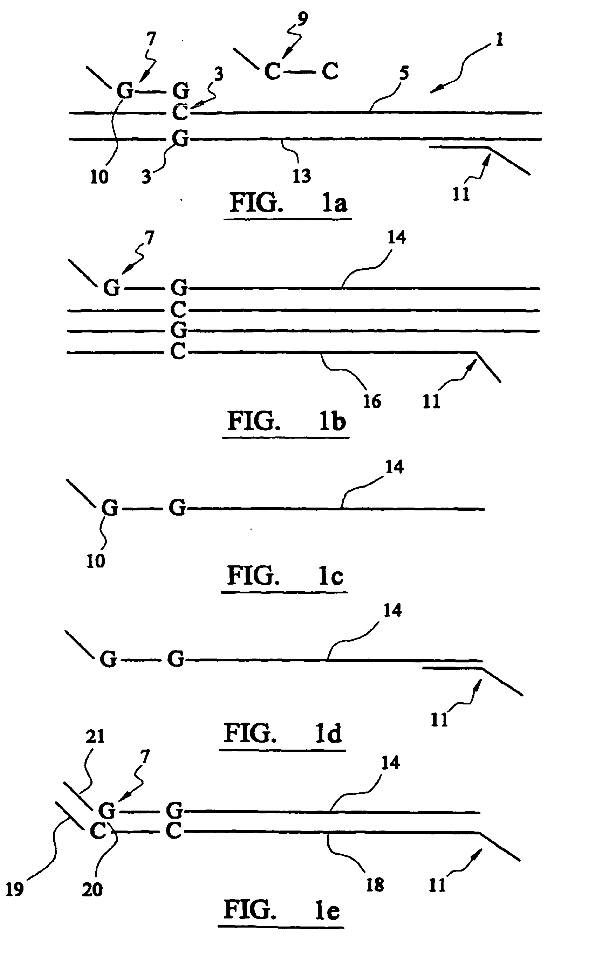

Figure 1 illustrates, according to one embodiment of the invention, a series of stages involved in the first amplification process based around a target template 1 with a potential C or G single nucleotide polymorphism 3 in one strand 5 of that target template 1. As illustrated in step A, the target template strand 5 of the particular individual under consideration has a C nucleotide at the SNP site 3.

The first step in this amplification stage involves contacting the template target 1 with two different forward primers 7 and 9, and a reverse primer 11. The forward primers 7 and 9 are locus specific primers, described in more detail below.

Forward locus specific primer 7 is terminated by a G nucleotide thus rendering it a match with the C nucleotide at the SNP site 3 and resulting in annealing of that primer 7 with the strand 5. The reverse primer 11 is non-specific and anneals to the other strand 13 of the template 1 at the appropriate location.

In step B, the specific forward primer 7 and the reverse primer 11 extend to produce the strands 14 and 16 through primer extension.

Denaturation of the strands results in the separation of the strands 5, 13 from their respective copied strands 14 and 16. The copied strand 14 only is shown in step C and the illustration of the subsequent steps.

Subsequent primer annealing, step D, is then performed again using the two forward primers 7, 9 and reverse primer 11. As we are considering strand 14 it is the reverse primer 11 which attaches to the strand 14 due to its sequence. The specific forward primer 7 would attach to strand 16, once again annealing in alignment with the site of the SNP 3 in that strand's sequence, not shown.

In subsequent primer extension, stage E, the reverse primer 11 extends the sequence of new strand 18 with the appropriate sequence given the sequence of strand 14, including the extension to produce tail portion 19 which arose as the strand 14 included the tail portion 21 of the forward specific primer 7. Due to the G base in the sequence of strand 14, the new strand 18 includes an opposing C base so as to match the identity of the SNP at site 3 in original strand 5. Due to the G base in the sequence of strand 14, due to the SNP related base 10, the new strand 18 includes an opposing C base 20 so as to match the identity of the SNP related site 10 in the originally copied strand 14.

Repetition of steps A through E over 20 to 25 cycles produces many millions of copies of sequences incorporating the same SNP identity, SNP repeat and surrounding sequence as the target template 1.

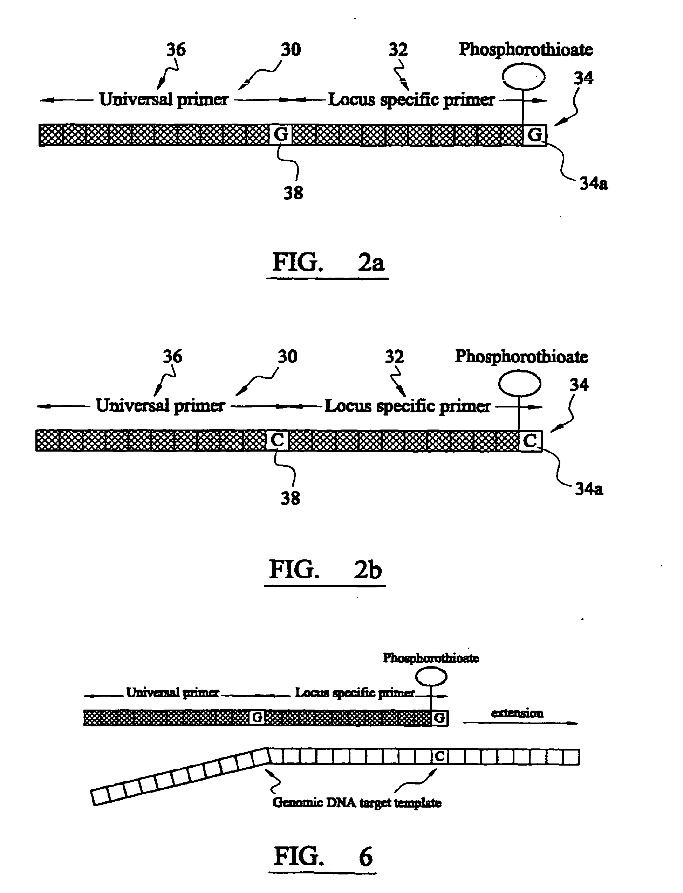

Figure 2a and b illustrate two locus specific forward primers, suitable for use in the stage detailed above, for use in investigating an SNP which could be either G or C. Each of the locus specific forward primers 30 consists of a locus specific portion 32 which has a sequence corresponding to the sequence of the loci under consideration up to the SNP site. The 3' end 34 of the locus specific forward primers ends in a G nucleotide 34a for one of the primers, Figure 2a, and in a C nucleotide 34b for the other primer, Figure 2b. Due to this different nucleotide used at the position corresponding to the SNP , then depending upon the identify of the SNP actually encountered, one of the locus specific forward primers will anneal thereto, but the other will not. Thus it is the forward primer of Figure 2a which anneals to the target in the example of Figure 1. This selectivity in annealing gives consequential specifity in the subsequent amplification cycles of the first stage.

In addition to the locus specific portion 32 the locus specific forward primer 30 includes a "universal" primer portion 36. The "universal" primer portion 36 consists of a nucleotide sequence which is identical for each of the two loci specific forward primers, save for a single nucleotide location 38 at the junction between the universal primer portion 36 and loci specific portion 32 of the primer 30. The nucleotide at the location 38 is identical to the 3' end nucleotide 34 of the locus specific portion 32 of the respective primer 30. Thus, the "universal" primer of Figure 2a incorporates G in its sequence at location 38 to reflect the G nucleotide present at the 3' end 34. The "universal" primer portion of Figure 2b, on the other hand, includes a C at location 38 to reflect the fact that a C nucleotide forms the 3' end 34 of this primer 30.

Whilst it is the locus specific portion 32 of the forward primers 30 which determines whether a primer anneals or not to the target, in the second and subsequent copying stages of the amplification process of stage 1, primer extension causes copying of the "universal" primer portion 36 of the primer sequence also and hence copying of the SNP equivalent nucleotide identity at location 38 too.

As previously stated the amplification process of the first stage results in a large number of copy sequences, including the SNP identity reflecting nucleotide and the matching nucleotide at location 38.

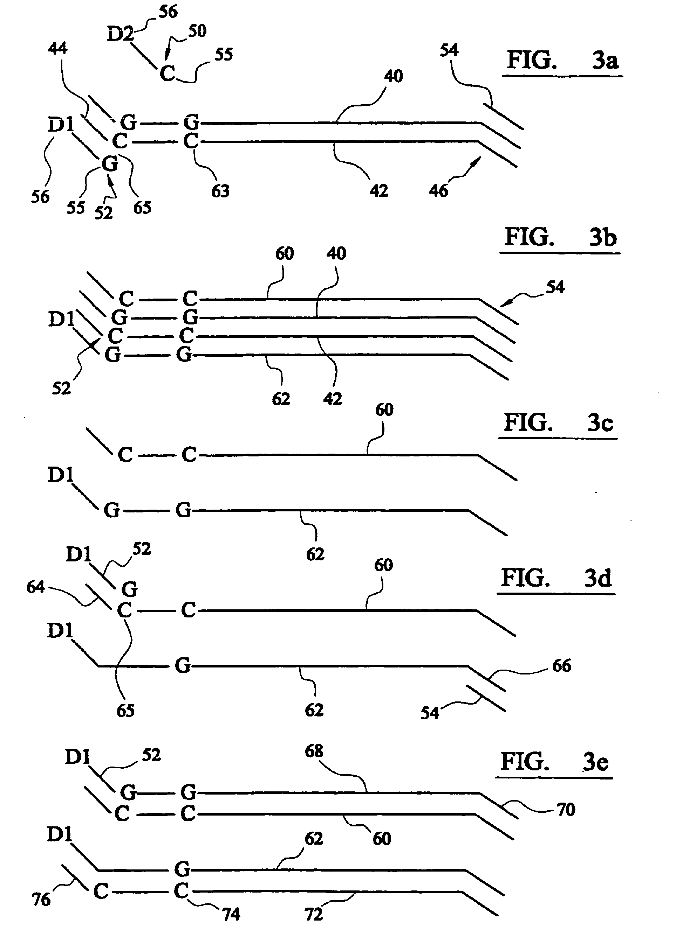

In the second stage of amplification, illustrated in Figure 3, a further specific amplification process is performed. It is much preferred that the second stage of amplification be conducted in the same vessel as the first, substantially simultaneous with the first amplification process. Such a possibility is described in more detail below.

For this stage, an aliquot of the amplification products from the first stage, described above, are taken and contacted with a pair of "universal" forward primers and a "universal" reverse primer. These "universal" primers are described in more detail below.

In step A, the strands 40 and 42 (copy strands which are equivalent to strands 14, 18 produced in the first stage as illustrated above) produced by the first stage 1 are denaturated and contacted with the two "universal" forward primers 50, 52 and reverse "universal" primer 54.

The two "universal" forward primers differ in terms of the 3' terminal end nucleotide 55 and in terms of a dye unit D or other form of label provided on the 5' end 56. The 3' end nucleotide 55 for the forward "universal" primers in this example is either C, "universal" primer 50, or G, "universal" primer 52.

As the strands 40 and 42 represent the outcome of copies of copies of the originals being taken, unlike strands 14, 18, they both have tail portions 44, 46 respectively which arise from the copying of the "universal" primer portions of the locus specific forward primer and reverse primer in the first stage.

The "universal" primers 50, 52 each have a sequence corresponding to the "universal" primer portion 34 of the first stage locus specific primers 30 up to location 38 of the locus specific forward primers 30. At location 55 the forward primers 50, 52 of the second stage have a base corresponding in identity to the identity of the nucleotide pairing to the SNP repeat in the stage 1 process, in one case, and in the other case corresponding to the identity of the other option for the SNP repeat. The nucleotide identity for the "universal" primers 50, 52 at location 55, corresponding to location 38, is thus different for the two primers 50, 52, with one providing one of the options and the other providing the other.

In the illustrated example, primer 50 carries a C and primer 52 carries a G nucleotide at position 55.

The sequence of the primers 50, 52 and particularly the identity at position 55 determines whether or not that primer 50, 52 anneals to the tail portion 44 of the strand 42 or not. In the illustrated case, strand 42 carries the SNP nucleotide C at site 63 as this was a copy of the identity of the SNP at site 3 in the original target strand 5. The C identity is also repeated in the tail portion 44 at site 65 as this was copied due to the copying of the tail of the original primer 7 by the reverse primer I 1 in the first stage. As a consequence the sequence of the tail portion 44 of strand 42 provides an annealing site for "universal" primer 52, but not primer 50. The reverse primer 54 anneals to the tail portion 46 of strand 40 due to the sequence matching.

Primer extension, step B, results in the production of strand 60 by matching strand 40, including SNP site copy C, and in the production of strand 62, including the match for the SNP, G, by matching strand 42 by the "universal" reverse primer 54 and specific "universal" forward primer 52 respectively. The SNP repeats are also copied.

Thermal denaturation is then used to separate the strands, step C, and from here on strands 60 and 62 only are considered although similar processes apply to the other strands too.

In annealing step D, the specific "universal" forward primer 52 anneals to the tail 64 of strand 60 due to the presence of a C nucleotide at the relevant position 65 in strand 60 and the consequential pairing to the "universal" forward primer 52. The reverse primer 54 anneals to the tail portion 66 of the strand 62.

In the further extension step E, the forward primer 52, which brings with it the label D1, extends the sequence of new strand 68, including tail portion 70. The reverse primer 54 extends the sequence of new strand 72, (thereby reproducing the SNP identity at site 74), including tail portion 76, (thereby reproducing the nucleotide corresponding to the SNP repeat 75 in that part too). Strand 62 already incorporates the label DI from its start as the primer 52 in step A

Once again, repeating stages A to E gives substantial amplification of the sequences and produces a great number of sequences label with a dye D1, the dye being selectively taken up as only one primer anneals and thus takes the dye into the sequence with it.

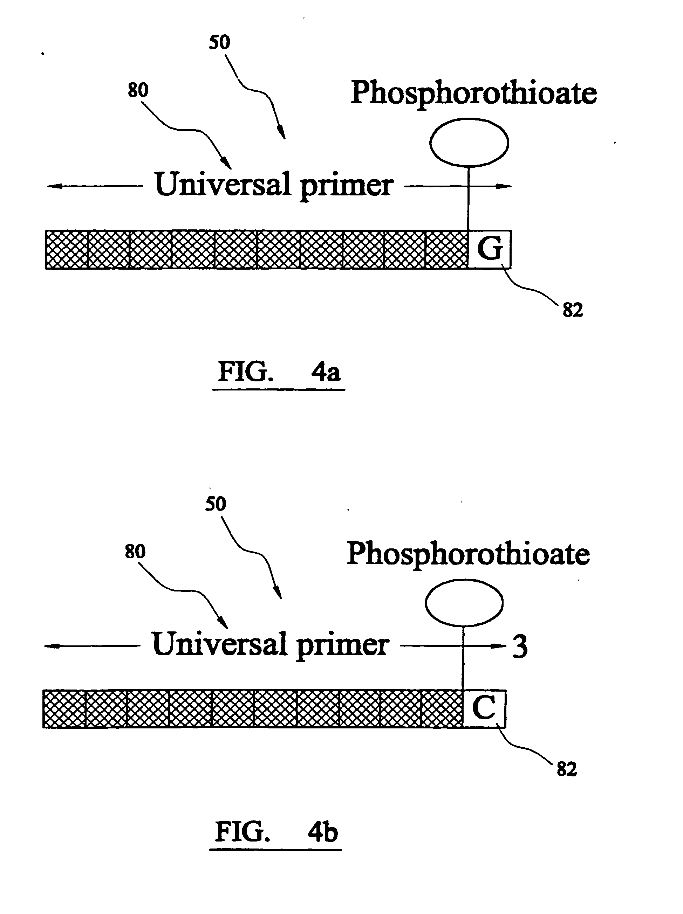

As described above, the second stage of the process uses a pair of "universal" primers on their own, illustrated in Figures 4a and 4b. These consist of a portion 80 having a sequence identical with the "universal" primer portion 36 of the locus specific primers 30 up to the single nucleotide variation at the end of the "universal" primer portion 36. The ends 82 of the universal primers of Figures 4a and 4b are different from one another and have an identity consistent with one of the two SNP possibilities, as is the case for the primers of Figures 2a and 2b. Thus, one "universal" primer 52, Figure 4a, is provided with G at its terminal 3' end 82 and the other "universal" primer 50, Figure 4b, is provided with C at its terminal 3' end 82.

During stage 2 of the process, these "universal" primers will selectively anneal to the amplification products of the first stage depending upon whether the tail portions extended and amplified during that stage incorporates the G or C variation.

Of course, equivalent primer types could be used with T or A variations in the above mentioned processes to investigate an SNP having potential T or A variation.

It is desirable for both the "universal" locus specific primers and the "universal" primers themselves to be provided with a phosphorothioate residue at the 3' end in order to protect the final base against exonuclease digestion by the Taq polymerase. This maximises the potential of the system to discriminate polymorphisms.

The different "universal" forward primers are provided with different labels/markers, in this case a JOE dye label and an FAM dye label respectively. The dye labels are provided at the 5' end of the forward primer in the second stage of the process. Of course, other different dyes and other forms of marking, such as radio nuclides could be used.

The "universal" primers were carefully designed to give desirable characteristics in terms of their melting temperatures, particularly a melting temperature of around 60°C. The sequences were also checked to ensure minimal hairpin formation and checked for minimal primer dimer formation. The sequences were also checked against human DNA sequence records and / or samples to ensure that human DNA is not amplified and to avoid any correspondence to any published sequence and particularly any part of the human DNA sequence. Primer dimer formation was also taken into account so as to keep such formation minimal.

An example of a suitable "universal" forward primer is provided (written 5' to 3') by the sequence :-
CGA CGT GGT GGA TGTG CTAR,
where R equals G or C or A or T depending upon the SNP to be detected for; and a suitable "universal" reverse primer is provided (written 5' to 3') by the sequence :-
TGA CCT GG CTG ACT CGA CTG.

The melting conditions for these primers, under 50mM primer and 50mM salt is 59°C with a GC content of 60%.

The specifity of the forward primers can be increased still further by making the ending for the primers of the first stage as follows, for a G detecting primer -TC and/or for a C detecting primer -AG and/or for a T detecting primer -CT and/or for a A detecting primer -GA.

By virtue of the different dyes or other indicators provided on the SNP specific "universal" forward primers an indication as to which of the possible SNP variations occurs at the SNP under consideration is given by the amplified product.

Although the technique has an advantageously low background noise at each locus, it is possible to incorporate one or more typed samples as controls.

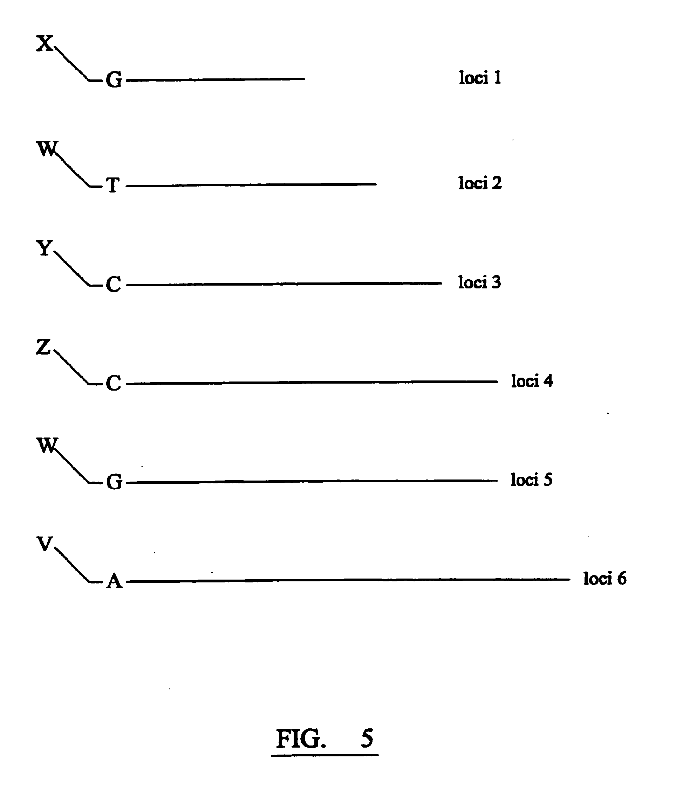

In Figure 5, the amplification products arising from a significant number of such a two stage processes are indicated. In this example, the sample subjected to the multiplex has produced amplification products with indications at:-
loci 1 with SNP G, indicated by dye X rather than dye Y;
loci 2 with SNP T, indicated by dye W rather than dye V;
loci 3 with SNP C, indicated by dye Y rather than dye X;
loci 4 with SNP G, indicated by dye Z rather than dye U;
loci 5 with SNP G, indicated by dye X rather than dye Y;
loci 6 with SNP A, indicated by dye V rather than dye W.

As the lengths of the sequences forming the amplification products at different loci are designed to be of different length, it is possible to separate those amplification products based on their size, for instance, using electrophoretic techniques and thus produce a series of lines on a gel whose colour is indicative of the SNP variation at the particular loci. Where the length of the sequence for one loci may be close to another, different dyes for each of the possibilities can be used. This in the example of Figure 5 loci I and 5 may have the same SNP variation but are sufficiently separable for the same dyes to be used. Loci 4 on the other hand is potentially close to loci 5 results and so different dyes for the results of the G, C variation for loci 4 and 5 are used.

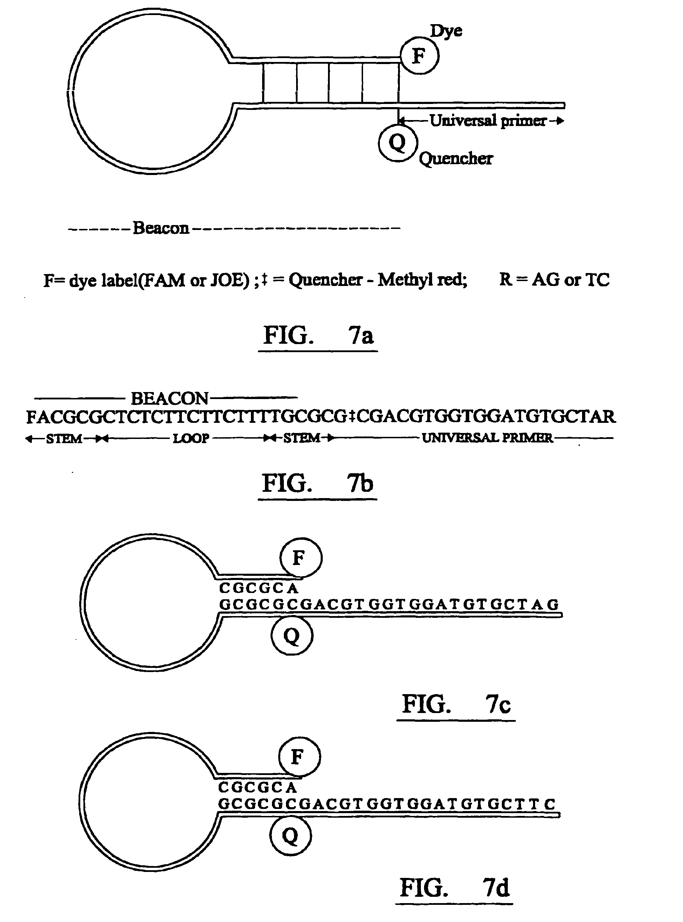

Figure 6 provides a detailed illustration of the locus specific primer annealing and subsequent extension where the SNP in the genomic DNA target template has a C SNP and as a consequence binds to the G incorporating locus specific primer.

As a further modification over the basic incorporation of a dye on the primer, it is possible to incorporate a so-called "molecular beacon". This consists of a single stranded DNA molecule that possesses a stem and loop structure. When the hairpin loop structure, illustrated in Figure 7a, is formed, the dye molecule is brought into close proximity to the quenching molecule, and as a consequence does not fluoresce when investigated. 5-carboxyfluorescin (FAM) offers a suitable fluorescent dye for use in such a situation with 4-4' dimethylaminophenylazo benzoic acid (DABCYL) offering a suitable quencher moiety. Methyl red could also be used as an alternative quencher, particularly branched methyl red.

A molecular beacon of this type, when the reverse primer extends into the beacon stem, becomes destabilised and gives rise to unfolding of the molecule. This moves the fluorescent dye label sufficiently away from the quenching molecule that quenching of the dye molecule no longer occurs. Subsequent investigation of the system would give fluorescence, therefore, the fluorescence being indicative of the particular dye and hence the particular SNP considered.

The particular sequence and components of the structure of Figure 7a are illustrated in Figure 7b. Further illustrations of universal primers in conjunction with molecular beacons are illustrated in Figure 7c, for a universal molecular G beacon with an AG sequence at its 3' end, and in Figure 7d for a universal molecular C beacon with a TC at its 3' end. The universal primer reverse sequence used with the universal molecular beacons of Figures 7c and 7d is:-

### TGC CGT GGC TGA CCT GAG AC

It is preferred that none of the universal molecular beacon incorporating primers contain phosphorothioate bonding.

A variety of ways of implementing the technique are possible, thus whilst PCR followed by direct inspection of the results by an instrument and / or inspection following electrophoretic investigations of the amplification products in gels could be undertaken, the technique is also suited to use with solid or other supports. Solid support media includes silicon, glass, plastics and magnetic beads. In particular, the technique is suited for implementation of the micro fabricated array system.

Micro fabricated arrays for implementing the present invention use oligonucleotides which are designed to terminate at the base which is penultimate to the SNP polymorphism under consideration. The oligonucleotides are attached to a solid support such as glass following a method, such as Z; Guilfoyle RA; Theil AJ; Wang R and Smith LM (1994) Direct fluorescence analysis of genetic polymorphisms by hybridization with oligonucleotide arrays on glass supports. Nucleic Acids Research (1994), vol 22: 5456-5465.

In such a method microscope slides are immersed in 1% 3-aminopropyltrimethoxysilane solution in 95% acetone/water for 2min. The slides are washed 10 times with acetone, 5 min per wash, dried for 45 min at 110C, treated for 2h with a solution of 0.2% 1,4- phenylene diisothiocyante (PDC) solution in 10% pyridineldimethyl formamide and washed with methanol and acetone.

These activated glass slides can be stored indefinitely in a vacuum desiccator.

The oligonucleotides are laid down in a grid pattern on the glass slide (using a robot it may be possible to lay down several hundred different oligonucleotides). The oligonucleotide contains a 5' end amino group introduced using the reagent - trifluoroacetyl-6-aminohexyl-2-cyanoethyl N', N'-diisopropylphosphoamidite. When the oligonucleotide is applied directly to the glass slide, the 5' end covalently binds to the glass.

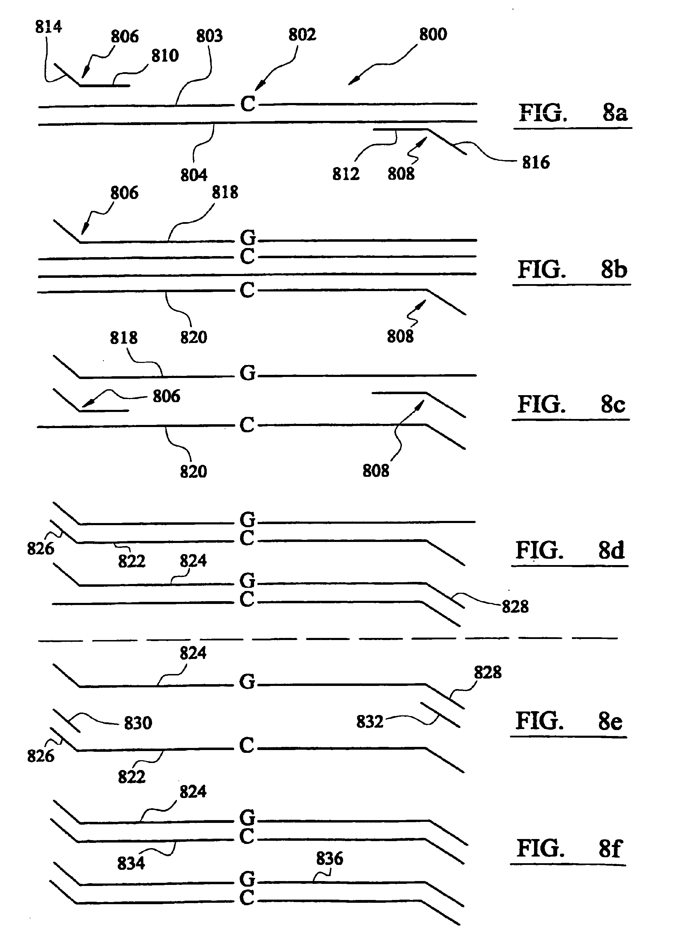

The microfabricated arrays can be used in a variety of ways, some of which are described in more detail below, and frequently in conjunction with amplified products produced according to the following technique. In the alternative technique, as shown in Figure 8a, a target sample 800 including SNP site 802, which has an identity of a C base, is under consideration in conjunction with corresponding strand 804. To achieve amplification a locus specific forward primer 806 and locus specific reverse primer 808 are introduced. Each of these primers is of the general type described above in comprising a locus specific primer portion 810 and 812 respectively and universal portion 814 and 816 respectively. The locus specific portions 810 and 812 are provided with sequences which match to sequences of the strands 803 and 804 respectively, but in contrast to the method described above, away from the site 802 of the SNP. Consistent with the format of the forward and reverse primers described above (see the description relating to Figures 2a and 2b, for instance), the universal primer portions have a sequence which does not hybridise to the locus in question and preferably does not hybridise to any naturally occurring DNA sequence.

Once hybridised, as shown in Figure 8b, extension causes the forward primer 806 to generate a copy strand 818 and causes the reverse primer 808 to form a copy strand 820. The strand 820 includes a base having an identity with the SNP and a strand 818 has a base pairing to the SNP identities.

Following denaturation of these strands, as shown in Figure 8c, strand 818 anneals with a reverse primer 808 and strand 820 anneals with a forward primer 806.

As illustrated in Figure 8d primer extension results in further copy strands 822, which includes a copy of the SNP identity and in terms of strand 824 which includes a copy of the base identity pairing to the SNP. These amplification products also include tail portions 826, 828 corresponding to the sequences introduced by the universal portions of the forward and reverse primers during earlier stages.

In the second amplification process of Figures 8e and 8f, which amplification can occur substantially simultaneously with the first stage and / or separately further amplification occurs. In this case, however, the amplification products for the first stage, strands 824 and 822 from the illustration of Figure 8d are contacted with forward and reverse primer. In this case, the forward primer 830 has a sequence corresponding to the sequence of the universal portion 814 of the forward primer 806 of the first stage. Similarly, the reverse primer 832 has a sequence identical with the universal portion 816 of the reverse primer 808 of the first stage. As a consequence of these sequences, the forward primer anneals to the tail portion 826 of strand 822 and the reverse primer 832 anneals to the tail portion 828 of strand 824.

Primer extension, Figure 8f, results in further copies of the strands 824, 822 being generated in terms of strands 834 and 836 respectively.

In the second stage of the process, as is true for the amplification process described previously, the fact that the amplification products from a large variety of loci will include the tail portions of the first stage primers means that a single forward and reverse primer can be used to further amplify all of the loci amplification products present, rather than having to use forward and reverse primers which are specific to the loci to achieve amplification. This renders the technique particularly suitable for amplifying a large number of target sequences from a large number of different loci simultaneously, without concerns as to coordinating melting temperatures and other properties.

The amplification products of the process described above can be analysed in a variety of ways.

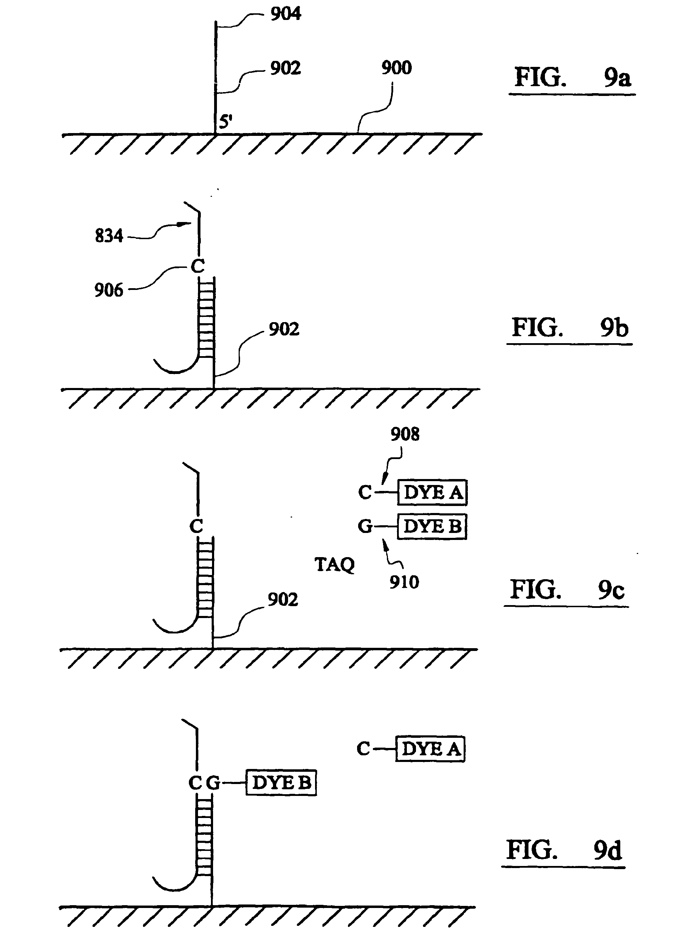

By way of example, and with reference to Figures 9a to 9d, it is possible to analyse these amplification products using genetic bit analysis. In this example, a solid support 900, such as glass, is provided for the array. Tethered to the array by its 5' end is a tailored oligonucleotide 902 which is designed to have a pairing sequence with the amplification products incorporating the SNP of interest. The tethered oligonucleotide 902 has a 3' end 904 which is one base short of the base which pairs to the SNP base.

In use, the amplification products are brought into contact with the oligonucleotides 902 of the array (substantial number of such oligonucleotides are provided with the illustrations only representing one such oligonucleotide for clarity). As a result of this contact, the amplification product, for instance represented by strand 834 from Figure 8f, hybridises to the tethered oligonucleotide 902 by virtue of the pairing sequence. As the oligonucleotide 902 is one base short of the SNP location 906, however, no base pairing to the SNP is provided during this hybridisation process.

In a subsequent step, 9c, dideoxynucleotides labelled with dyes are introduced. In the Figure 9c example a dideoxynucleotide 908 is provided labelled with a dye A and a dideoxynucleotide 910 is provided with a dye B. Dideoxynucleotide 908 is a C base, whereas dideoxynucleotide 910 is a G base. Addition of these dideoxynucleotides in conjunction with TAQ results in the TAQ polymerase adding the appropriate base on to the tethered nucleotide 902 as shown in Figure 9d. In this case, as the base pairing to the SNP is a G base, it is dideoxynucleotide 910 which is added and as a consequence, with it, dye B.

In a subsequent stage, not shown, the un-fixed dideoxynucleotides and their dyes are washed from the micro-fabricated array and inspection of the array reveals that it is dye B and as a consequence dideoxynucleotide 910 which has been added to the tethered nucleotide 902 and that as a consequence the identity of SNP 906 is a C base.

Investigations for other bases can be achieved simultaneously using different dyes for each of the four possibilities and with different tethered oligonucleotides being provided at different locations in micro-fabricated arrays. Thus a substantial number of loci can simultaneously be investigated using loci specific tethered nucleotides in conjunction with a different dye attached to each of the four possible dideoxynucleotides.

The method of Genetic Bit Analysis (GBA) is used to detect the polymorphism, as described in Nikiforov TT; Rendle RB; Goelet P; Rogers YH, Kotewiez MI, Anderson S, Trainor GL, Knapp MR (1994) Genetic Bit Analysis: a solid phase method for typing single nucleotide polymorphisms. Nucleic Acids Res 22:4167-75.

In the hybridization solution, there may be 4 dideoxy bases (A,G,C,T) which are dye-labelled with different dyes e.g. TAMARA, JOE, FAM, HEX. Taq or Klenow polymerase may be included in the reaction mix. This extends the oligonucleotide by just one base, with the complementary dideoxy - which subsequently fluoresces a colour which is dependent upon the complementary base.

As an alternative to genetic bit analysis a ligation assay can be used using such arrays. To investigate the identity of the SNP in the type of amplification product produced by the process described above in relation to Figures 8a to 8f. In this method, tethered oligonucleotides 1001 are provided on the support 1003 and micro-fabricated array is contacted with the amplification products.

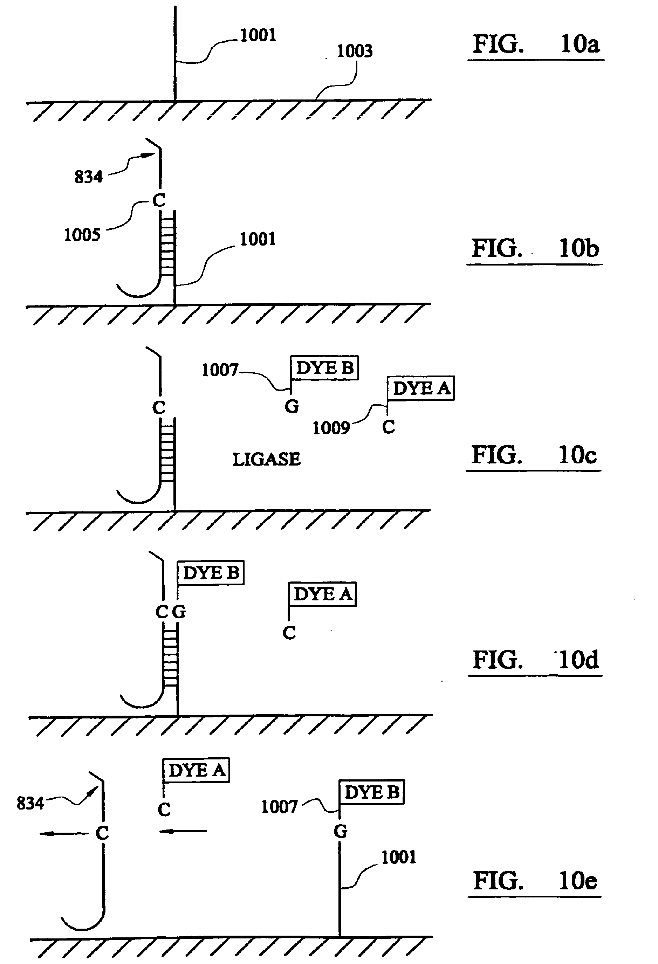

As the tethered oligonucleotides 1001 are provided with a sequence which pairs to the sequence of the SNP incorporating strands, for instance strand 834 from Figure 8f, that strand hybridises to the tethered oligonucleotide 1001. The sequence of the tethered oligonucleotide 1001 stops one base short of the SNP site 1005. As a consequence, as shown in Figure 10b, no base pairing to the SNP site 1005, in this case base C, is provided.

In the subsequent step illustrated in Figure 10c to allele specific primers are brought into contact with the tethered oligonucleotide 1001 and the hybridised strand 834. These primers are provided with 3' end bases representing the possible base identities which would match with the SNP base. Thus, oligonucleotide 1007 is provided with a 3' end G base and oligonucleotide 1009 is provided with a 3' end C base. The oligonucleotides 1007 and 1009 also incorporate different dye molecules from one another. By providing these oligonucleotides 1007 and 1009 together with ligase then only the oligonucleotide 1007 or 1009 which has a 3' base pairing to the SNP base identity will be introduced to the end of the tethered oligonucleotide 1001, see Figure 10d.

Following the successful ligation, as illustrated in Figure 10e, the temperature is raised to melt away the oligonucleotides carrying the unincorporated dyes and the amplification product, strand 834 itself. As a consequence, only the tethered oligonucleotide 1001 and oligonucleotide 1007 incorporating its dye remain. Investigating the colours present for the various arrays of the micro-fabricated array then reveals the identity of the dye and hence the 3' base identity for the oligonucleotide and hence the base identity and the SNP 1001 for each of the amplification products under consideration.

Again, a substantial number of loci can be considered simultaneously by the use of different tethered oligonucleotide 1001.

The basic technique of the invention could also be used in conjunction with mass spectrometry and/or micro titre plate based assays, including Taq-man assay and molecular beacons.

To improve the technique's ability to distinguish accurately SNP differences following amplification, the following modifications can be made. In the techniques described above the "universal" primer portion of the forward primers of the first stage add equivalent sequences in each cases. The only difference was in the single nucleotide identity located at the junction between the universal primer portion and the loci specific portion of the primer. In this modification, however, the universal primer portion of the forward primer is provided with a distinct sequence in each case when compared with other forward primers for different SNP identities. Given that there are only a maximum of four possible SNP identities, a maximum of four different forward primers, each having a different universal primer portion are required for any one SNP. The reverse primer also includes a universal primer portion having a different universal primer portion to the portions of the forward primers. The function of the reverse primers and forward primers during amplification is equivalent to that described above.

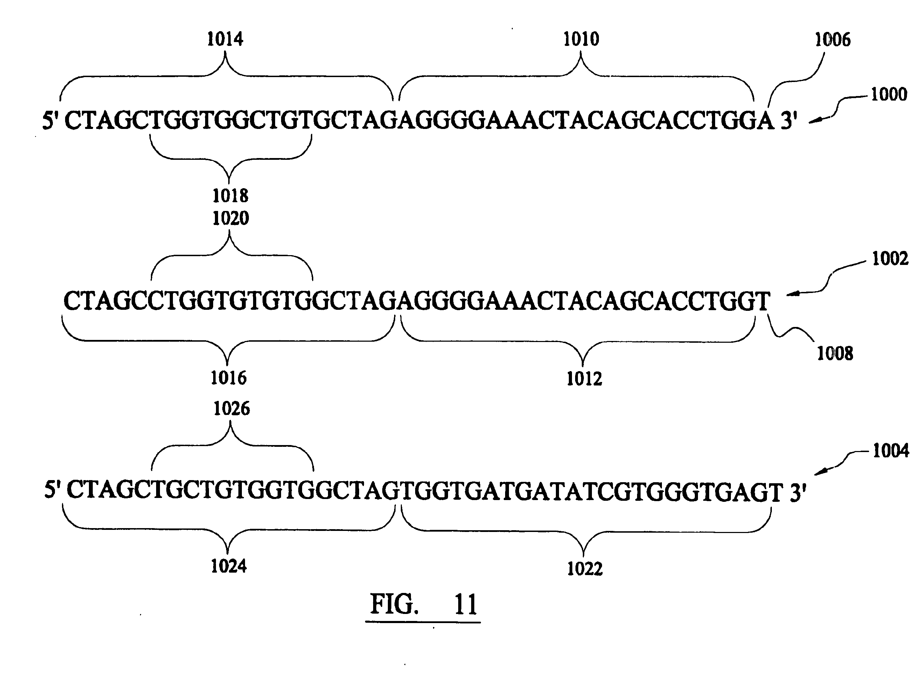

Examples of suitable forward primers, 1000 and 1002, are illustrated in Figure 11 together with a suitable reverse primer, 1004. The forward primer 1000 is locus specific to alpha-1-antitrypsin Ml/S polymorphism with the aim of detecting a T based variation whereas the forward primer 1002 is intended to detected an A based variation at the SNP. This is reflected in the identities of the SNP identifying portion 1006 and 1008 respectively. The locus specific portion, 1010, 1012 respectively, of these two forward primers, is attached to a universal primer portion 1014, 1016 respectively, with the universal primer portion incorporating a distinguishing portion 1018, 1020 respectively in each case. In this example the distinguishing portions are provided within the overall sequence of the universal primer portion for reasons described in more detail below. However, the entire universal primer portion or one end of the universal primer portion could be used to provide the distinguishing sequence.

A similar structure is provided for the first primer in terms of locus specific sequence 1022, universal primer portion 1024 and distinguishing portion 1026.

The universal primer portions are preferably different from one another by at least 6 bases. Universal primer portions having a sequence difference of between 25 and 100% when compared with one another is advantageous.

One of the principal advantages of this alteration to the universal primer portion sequence is that it introduces multiple base changes into the amplification products, and as a consequence renders the amplification products more suitable for investigation and detection using hybridisation on to solid support surfaces, such as glass. This technique is described in more detail below.

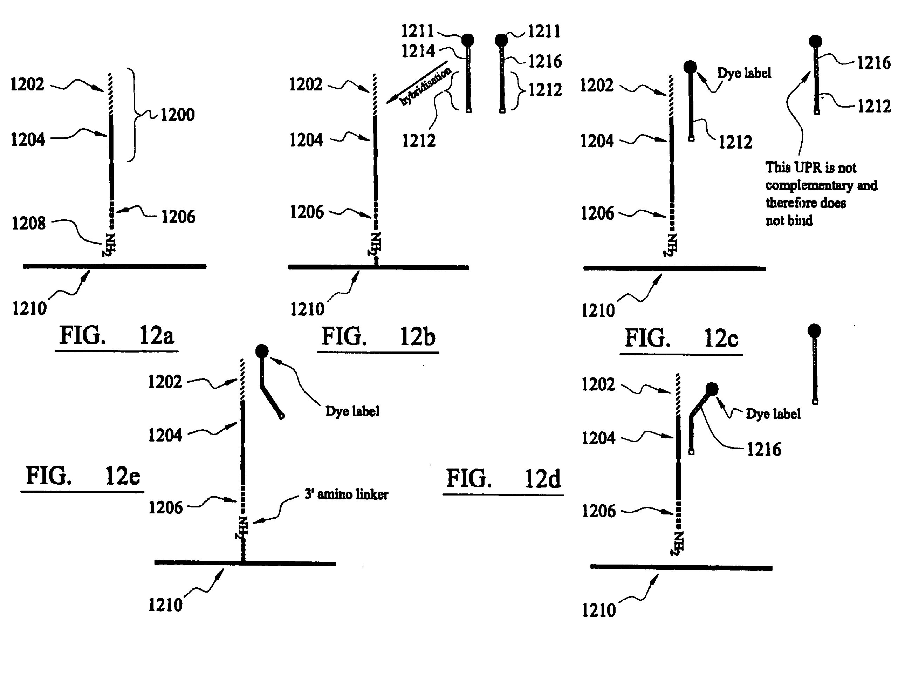

Having produced amplification products which are substantially different in terms of their bases from one another, the amplification products are particularly suited to investigation and analysis using the type of technique illustrated in Figure 12.

As illustrated in Figure 12a, the amplification product consists of the forward primer sequence 1200, including the universal primer portion 1202 and locus specific portion 1204 and the reverse primer portion 1206. By providing a amino end group 1208 on the terminal 5' end of the reverse primer the strand can be covalently attached to an epoxy-silane treated glass slide, 1210. The preparation of such epoxy-silane slides is based on the method of Beatty et al, Molecular Biology, Volume 4, 1995, 213-225.

Other attachment chemistry, for instance the use of 5' ends labelled with phosphorothiate can be used to attach such strands to bromo-acetamide slides for instance.

Generally, the amplified product is purified for instance using a centricon filtration to remove an incorporated primer and dNTP's. It is then extracted with water before spotting on to the glass slides, for instance using an Amersham generation 3 micro array spotter. The slides can be incubated in an high humidity chamber for between 30 minutes to two hours at 20°C to 40°C before washing in water at 95°C, 10mM triethyomine (pH 9.2) at room temperature, and two further washes with water at 60°C before being stored dry at room temperature.

To effect the detection step the single strands must be contacted with suitable fluorescent probe constructs. In Figure 12b a biallelic system is being introduced, and as a consequence two different fluorescent probes are introduced. Each fluorescent probe has a different dye label 1211, common locus specific portion 1212 and different universal portions 1214 and 1216 respectively. Hybridisation is carried out at low temperature, 40°C. Probe specifity is controlled by carrying out post-hybridisation washes at higher temperatures.

As illustrated in Figure 12c only one of the fluorescent probes is sufficiently complimentary to hybridise completely to the fixed strand. By conducting the post-hybridisation washes at a sufficiently high temperature (60°C to 75°C) specifity of the probes is maintained as at such temperatures the fact that the locus specific portion 1212 of the other fluorescent probe corresponds is insufficient to remain hybridised due to the difference between the universal primer portion 1202 and 1216, Figure 12d. As a consequence the second fluorescent dye label is not retained by the single strand.

On a similar basis, Figure 12e, if the universal primer portions are complimentary, but the locus specific region is different, a fluorescent probe for a different locus, hybridisation cannot occur sufficiently for the fluorescent probe to be retained on a similar basis.

Experimental conditions for such a system are described below.

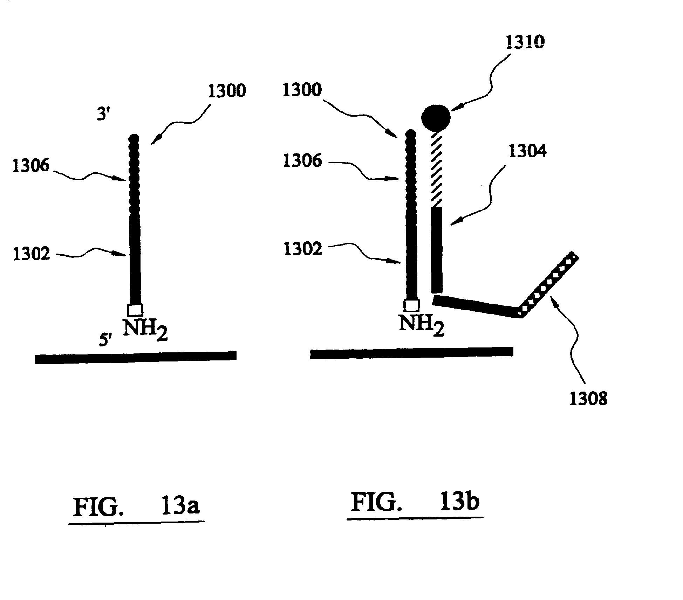

As an alternative analysis technique, synthetic oligonucleotides can be spotted on to the slide and covalently bound thereto using an amino-linker at 5' end. Such a process is illustrated in Figure 13a. Once contacted with the amplification products in cases where the oligonucleotide 1300 has a sequence for its locus specific portion 1302 which matches the locus specific portion of the amplified product 1304 and the universal primer portion 1306 matches that amplified product then hybridisation will occur. The universal reverse primer sequence 1308 does not participate in the hybridisation. Again the dye label molecules 1310 can be inspected in such cases to indicate the identity.

### EXAMPLE - Multiplex amplification

As well as the above mentioned two distinct stage process, a single vessel process is also envisaged. This involves adding all first reaction primers at a concentration of 50nM with the exception of any primers for which there would be competition, for instance due to primer site overlap. This is exemplified by forward primers Gc 420G/T and 416C/A for instance of the specific primers identified. When competition potentially occurs it is necessary to balance the reaction by trial and error experimentation of the concentrations. For the above mentioned specific examples the optimum concentration for the Gc 416C/A primers were 25nM and the 420G/T primers were at 50nM. The reverse primer optimum concentration was 100nM for all cases.

To this mixture the universal primers used in the second stage are then added. As a rule of thumb, the amount of universal primers used must be increased according to the number of first reaction primer sets utilised. The concentration (Cn) of each universal primer is Cn x L where L is the number of loci (or SNPs) analysed. Thus for a 5 locus system the concentration of each of the universal primers is 50 x 5 =250nM. The amount of DNA is held constant at 1 ng for optimum amplification but it is possible to analyse lower quantities of DNA. At higher numbers of loci it is believed that the concentration of primer which is usefully deployed will plateau out.

The overall combination was then subjected to the amplification process as follows:-
Summary of conditions:
   50ul PCR reaction total volume.
   Buffer = Perkin Elmer PCR buffer
   MgCI₂ = 1.5mM
   DNTPs = 200uM each
   Taq Gold (Perkin Elmer) 1.25units
Cycling conditions:
   94°C for 30 seconds
   61 °C for 30 seconds
   72 °C for 90 seconds
   6 cycles

   94°C for 30 seconds
   72-75°C for 30 seconds
   for 5 to 10 cycles

   94°C for 30 seconds
   61°C for 30 seconds
   72 ° C for 90 seconds
   for 24 to 29 cycles

The resulting amplified products were then considered in the manner outlined above, electrophoresis based separation for instance, to determine which label and hence which SNP were present at each loci under consideration.

The underlying technique of this invention offers a significant number of advantages:-
1) the universal primers themselves do not prime human DNA, thus artifacts mediated by mis-priming of degraded DNA are minimal and the pull-up artifact is easily recognised since the electrophoretic migration rates of different SNP's is different;
2) the use of a two stage reaction process improves specifity;
3) mis-priming is virtually negligible, thus virtually eliminating background noise and rendering the technique useful for very low level mixtures;
4) the need for dye or other labels on the universal primers only significantly reduces production costs for the reagents;
5) large numbers of different loci may be amplified in the second stage of the process using only one set of primers thus rendering large number multiplexing possible;
6) the use of only two forward primers in the second stage gives a balanced reaction without competition between primers;
7) the use of a two stage process enables very small levels of feed material to the first stage, sub-nanogram levels, to be amplified to the extent where several aliquots are produced for use in potentially different second stage processes.
8) by providing a one tube reaction the conduct of the reaction is simplified and speeded up.

These and other advantages, and features of the present invention are apparent from the following practical demonstrations of the invention.

### EXAMPLE - Dye based

### Mitochondrial DNA

Tully et al (1996) Genomics 34, 107-113, described a minisequencing approach to analyse mitochondrial DNA SNPs. The SNPs listed in table 1 were analysed using the approach described above but with the primers amended according to the present invention to provide universal G or universal C attached to the 5' end of the primers listed.

The sizes of each DNA fragment are known and when run on a gel, bands indicative of a loci are produced and these are either JOE (green) for universal primer E or FAM (blue) for universal primer C labelled depending on the SNP identity, so allowing visualisation of the results.

**TABLE 1**

| **Forward Primers Position** | **Primer Sequence Used With Universal C** | **3' Polymorphism Used With Universal G** |
|---|---|---|
| 73 | GTATTTTCGTCTGGGGGGTA | G |
| 146 | GTCTGTCTTTGATTCCTGCCC | T |
| 152 | TTTGATTCCTGCCTCATCCC | T |
| 195 | ATATTACAGGCGAACATACC | T |
| 247 | GCTTGTAGGACATAATAATAACAATT A | G |
| **Reverse Primer** 326 | CAGAGATGTGTTTAAGTGCTGT | |

### Reaction conditions:

For each separate reaction:
DNTPs were at a final concentration of 35mM
Perkin Elmer (PE) buffer was at a final concentration of 0.375mM with 0.375mM MgCl₂.
0.25 AmpliTaq (PE) was added to 50ul reaction.

Primer concentrations are detailed separately with examples given.

All phenotypes were verified by independent analysis using the mini-sequencing method of Tully et al (1996).

### Example 1

### Multiplexed mitochondrial DNA

### Reaction conditions:

DNTPs all at 10mM;
Final concentration of 35mM.. PE buffer 15mM 15mM MgCI2 per reaction MgCI2 =.375mM. AmpliTaq =.25ul in 50ul

In the following example, 1 uM of each of the forward primers and 2uM of the reverse primer listed in table 4 was used in the reaction mixture. A 50ul reaction containing 0.3ng of genomic DNA was amplified through 8 cycles at 94C for 30sce; 57C for 30sec and 72C for 90sec. An aliquot of 5ul of the reactant was then transferred into a second tube containing 1uM of each forward universal primer and 1uM of the reverse universal primer. This was amplified for 22cycles at 94C for 30sec, 62C for 30sec and 72C for 90sec. Samples were electrophoresed on a ABD 377 automated sequencer with Rox 500 sizing standard. The negative control was treated under the same conditions, except that no DNA was added to the reaction.

The results are illustrated for the four samples in Figure 11, with each of the three samples being represented by one trace and with the control sample (no DNA added) being the fourth trace. The peaks labelled a are size standards, those labelled b indicated green labels and those labelled c indicating blue labels. These samples were analysed against 4 mitochondrial loci, 247, 195, 152, 146. The identities revealed for the four loci are summarised in Table 4.

**Table 4**

| Sample | Loci 247 | Loci 195 | Loci 152 | Loci 146 |
|---|---|---|---|---|
| One | G | T | T | T |
| Two | G | C | C | C |
| Three | G | T | C | C |
| Control | none | none | none | none |

### Example 2

### Elucidation of a mixture where the minor component is <10pu DNA (genomic equivalent).

In the next example, the results for which are illustrated in Figure Y2, mixtures were prepared with the major component coding for the mt0073A polymorphism (2ng genomic DNA) and the minor component coding for the mt00326 polymorphism (0-50pg). Amplified with forward primers, either mt0073-G or mt0073-A (luM) and the reverse primer mt00326 (luM), the cycling conditions were the same as described previously but the second round amplification was just 3 cycles.

In the first experiments, left hand series, (a) primers used were mt0073-G (luM) and mt00326 (luM) whereas in experiments, right hand series, (b) primers were mt0073-A (luM) and mt00326 (luM). The results (a) showed that even in the presence of very great excess of mt0073-G template, there was no mt0073-A background product detected. Similarly, in experiment (b) using just primer m00O73A there was no mt0073-G detected. The high specificity of the reaction demonstrated discrimination of minor components in mixtures down to extremely low levels of 12,5pg in a total - a mixture ratio of 1:200.

In the figures, peaks a are size standards, peaks b indicate a green response and peaks c indicate a blue response.

### Example 3

### Genomic DNA - Group Specific Component (Gc)

The Gc single nucleotide polymorphisms have been well characterised (Braun et al, 1992) Hum Eanet, 89:401-406. In addition a large number of rare variants have been identified - the test described here only detects the common alleles - Gc 2, Gc1F and Gc1S. Reynolds and Sensabaugh (1990) in Polesby et al (eds) Advances in Forensic Haemogentics, Vol. 3 Springer, Berlin, Heidelburg, New York pp 158-161 compared cDNA sequences of Yang et al (1985) Pioc - Nat - Accad - Sci USA 82:7994-7998 and Cooke N. E. and David E. V. (1985) Serum D - binding protein is a 3^{rd} member of the albumin and alpha-feto protein gene family, J. Clin. Invest. Vol. 76 pg. 2420 - 2429. Although polymorphisms were observed at 4 different sites, the most informative are at codons 416 and 420, where single base changes result in an amino acid change. At triple 416, GAT codes for an aspartic acid residue in the Gc2 and Gc1F phenotypes, whereas GC1S has a glutamic acid residue determined by codon CAG. Amino acid 420 is a lysine residue in the Gc2 phenotype coded by AAG; a threonine residue in both Gc1 phenotypes is coded by ACG.

Four different forward primers were prepared to distinguish between the various polymorphisms (table A, B). These primers were attached at the 5' end to universal primers as described previously.

| **codon** | **sequence** |
|---|---|
| Forward primers 420G/T 416C/A | ACCAGCTTTGCCAGTTCCR TTCCGTGGGTGTGGCX |
| Reverse primer | GGCAGAGCGACTAAAAGCAA A |

**Table A: Sequence of primers used to detect Gc1F, Gc1S and Gc2 polymorphisms. R=G or T; X=C or SA. 420T and 416A were attached to FAM labelled universal primer G; 420G and 416C were attached to JOE labelled universal primer C.**

| | | | |
|---|---|---|---|
| 416 | | 420 | |
| | | G | T |
| | A | Gc1F | Gc2 |
| | C | Gc1S | |

### Table B: The GC phenotypes are dependent upon the codon mutations detected. Note that 416A and 420T do not exist together in coupling. The 420G primer detects Gc1 phenotypes; 420T detects Gc2; 416C detects Gc2 or Gc1F (dependent on codon 420 sequence); 416A detects Gc 1 S.

Using these details a series of examples were undertaken with two aspects being tested, specificity, and sensitivity. To carry out specificity tests, a series of singleplex reactions were carried out. In Figure 12a primer 416A was shown to be a specific test for the Gc1F polymorphism. Similarly, primer 420G was specific for Gc1 polymorphisms (Figure 12b); 420T was specific for Gc2 polymorphisms (Figure 12c). The system was demonstrated to work with all primers in a cocktail mix, Figure 12d. Furthermore sensitivity of detection was c. 8pg genomic DNA (Figure 12d). A mixture was analysed this demonstrated that mixtures are easily interpreted, the different molecular weights of FAM and JOE confer different molecular weights on the DNA fragments the same size, and this facilitates interpretation. It is easy to distinguish the artefact pull-up from a true allele.

### Reaction conditions

The reagent concentrations were the same as described for mitochondrial DNA. Primer concentrations used were 125nM for the locus specific forward primers and the reverse primer. The universal forward primers were at 100nM, and the universal reverse primer at 288nM. Locus specific and universal primers were admixed in a single tube reaction. The cycling conditions used were 94 C for 30 sec; 61 C for 30 sec; 72 C for 90 sec for 35 cycles, followed by 72 C for 10 min.

All phenotypes were verified by independent analysis using conventional isoelectric focussing.

### EXAMPLES - Molecular beacon based

To demonstrate the benefits of the universal primer incorporating beacons, a series of experimental designs were investigated using a Roche light cycler to analyse the fluorescence resulting. The experiments were performed using a two-tube or branched PCR approach to give first and second round amplifications.

The following protocol for amplification in both the first and second round PCRs was used for the various examples which follow.

### 1^{st} Round PCR

| **Primer** | **Optimum Concentration** |
|---|---|
| 1^{st} round forward primer | 200nM |
| 1^{st} round reverse primer | 200nM |
| MgCl₂ | 1.5mM |
| BSA | 0.2µl/reaction |
| PE Buffer | 10% |
| d NTP's | 200µM |
| Taq Gold | 0.1µl/reaction |
| DNA | 2ng/reaction |
| SDW | |
| | |
| Reaction Volume = 20µl | NB Final [MgCl₂] =3mM |

1^{st} round amplification was carried out as follows:-

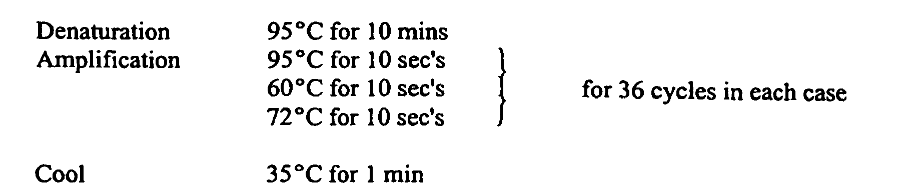

### 2^{nd} Round PCR

| **Primer** | **Optimum Concentration** |
|---|---|
| 2^{nd} round forward beacon | 200nM |
| 2^{nd} round reverse primer | 200nM |
| MgCl₂ | 1.5mM |
| BSA | 0.2µl/reaction |
| PE Buffer | 10% |
| d NTP's | 200µM |
| Taq Gold | 0.1µl/reaction |
| 1^{st} round PCR product | 2µl/reaction |
| SDW | |
| Reaction Volume = 20µl | NB Final [MgCl₂] = 3mM |

2^{nd} round amplification was carried out as follows:-

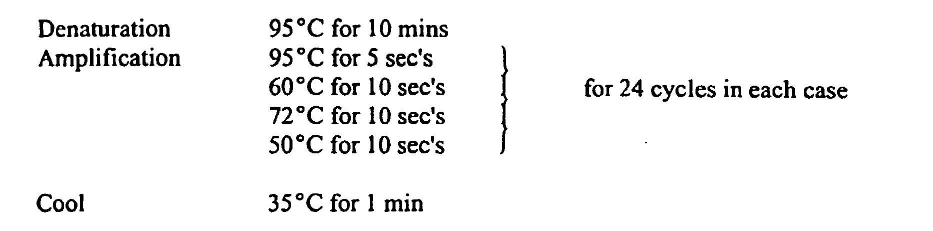

A reading of fluorescence was taken at the end of each 50°C hold.

### Example A

In this example, DNA was amplified with primers directed towards the Gc1s polymorphism in the 1^{st} round. Subsequent 2^{nd} round PCR, amplification was directed to this 1^{st} round product using increasing concentrations, (200nM, 400nM, 600nM, 800nM and 1000nM), of 2^{nd} round primers. The respective first round forward universal primers and reverse primer and second round universal primer incorporating a molecular beacon and universal reverse primer are as listed below. The stem part of the molecular beacon is underlined in the universal primer sequence incorporating the molecular beacon.

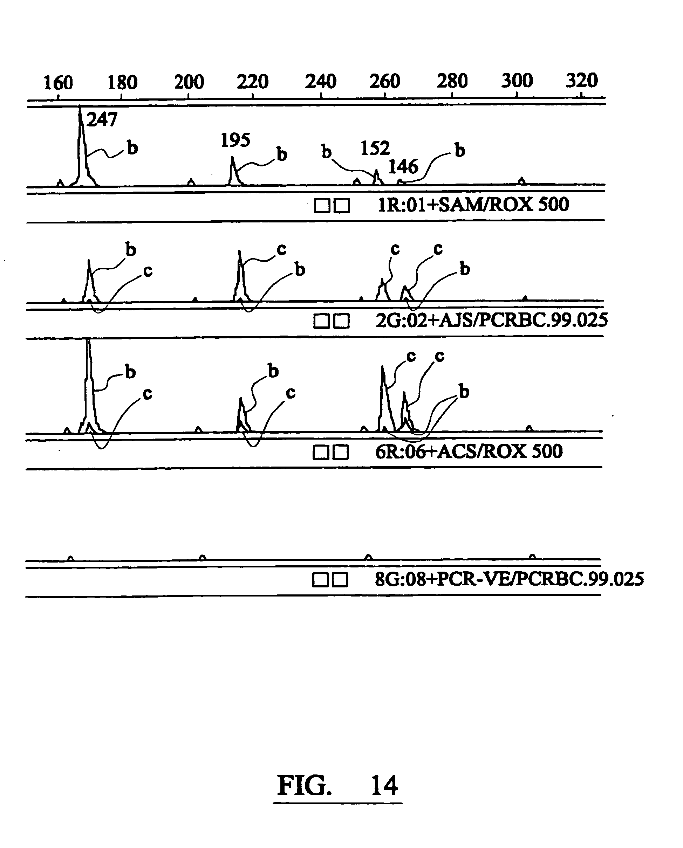

The results, displayed as a graph of fluorescence against 2^{nd} round amplification cycle number for the various concentrations of 2^{nd} round primer and a Gc1s polymorphism 1^{st} round universal primer are displayed in Figure 14. Very substantial discrimination between the polymorphism featuring samples and the controls can be seen.

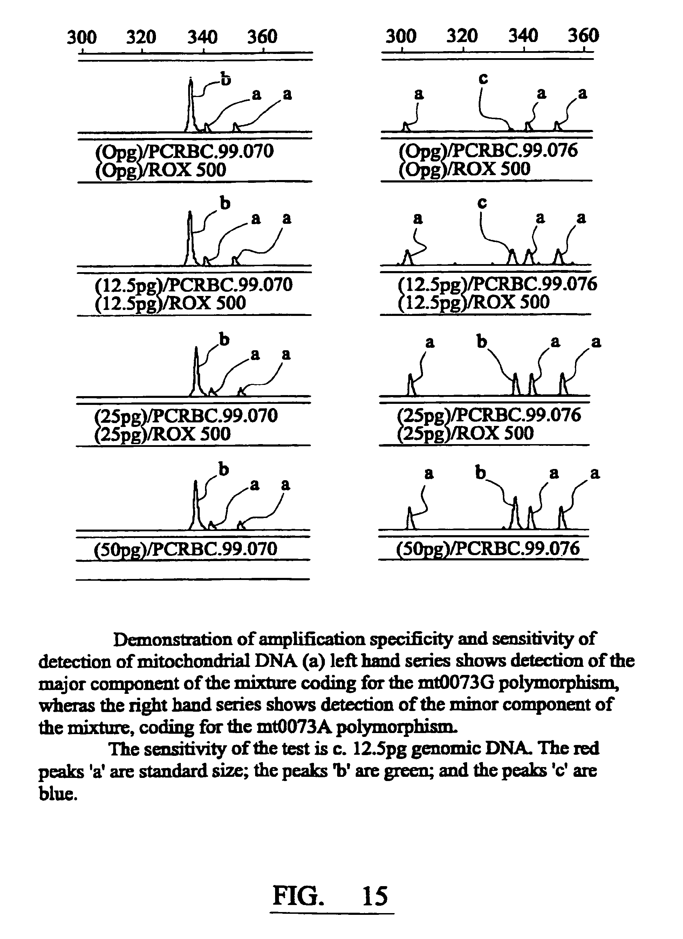

In order to confirm the finding that the universal primer beacons amplify DNA specifically all PCR products were run on 3% Nusieve minigel. As can be seen from the typical results set out in Figure 15 for the minigel image, no product is seen for the SDW control and two bands are present for each DNA sample, 1a and 1b. The upper band sizes is at 112bp which is consistent with Gels s amplified product. The additional band is approximately 12bp smaller and is thought to be the result of single stranded 2^{nd} round product where the beacon has adopted its intra molecular secondary structure. However, this additional band does not interfere with interpretation and appears not to compromise the PCR in any way.

### 1^{st} round primers

| **Primer name** | **Primer sequence** |
|---|---|
| Gc1s uni 9 G | CGACGTGGTGGATGTGCTAGGTTCCGTGGGTGTGGCC |
| Gc reverse uni 11 | TGACGTGGCTGACCTGAGACGGCAGAGCGACTAAAAGCAAA |

### 2^{nd} round primers

| **Primer name** | **Primer sequence** |
|---|---|
| Universal G beacon | F-ACGCGCTCTCTTCTTCTTTTGCGCG-Q-CGACGTGGTGGATGTGCTAG |
| Universal 11 reverse | TGACGTGGCTGACCTGAGAC |

### Example B

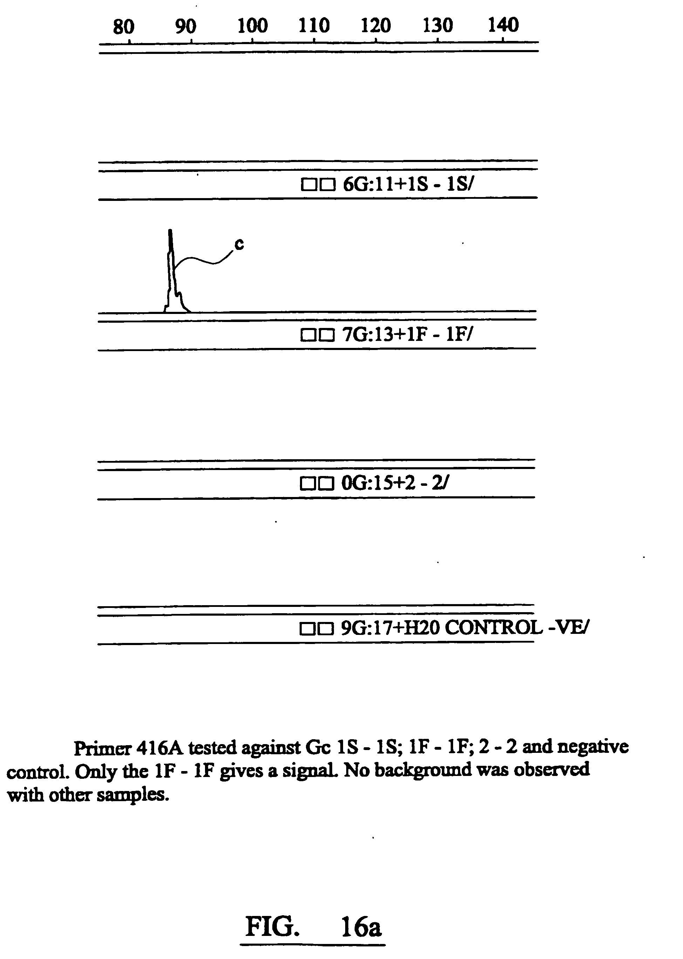

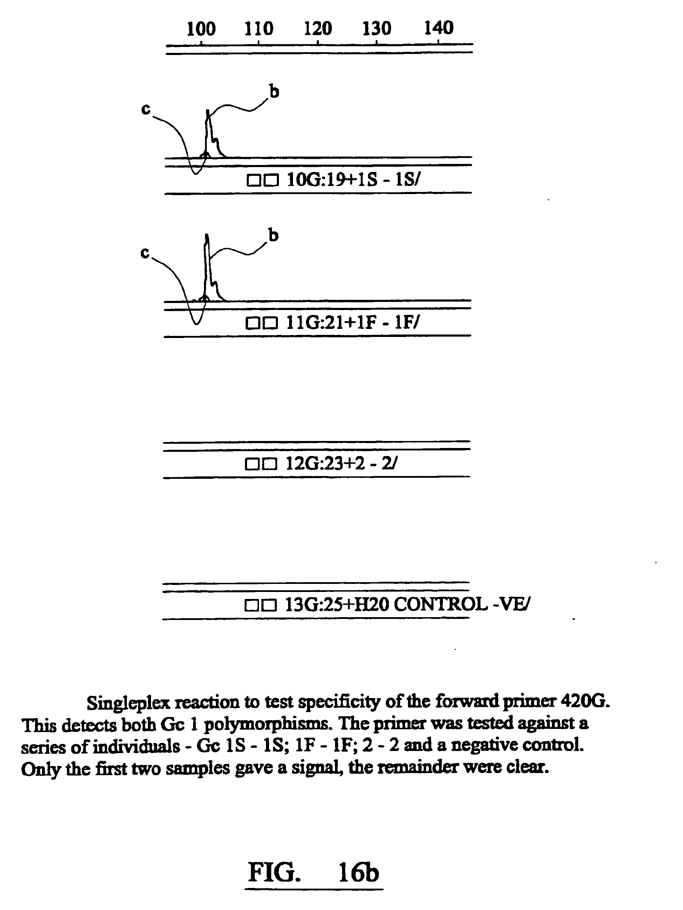

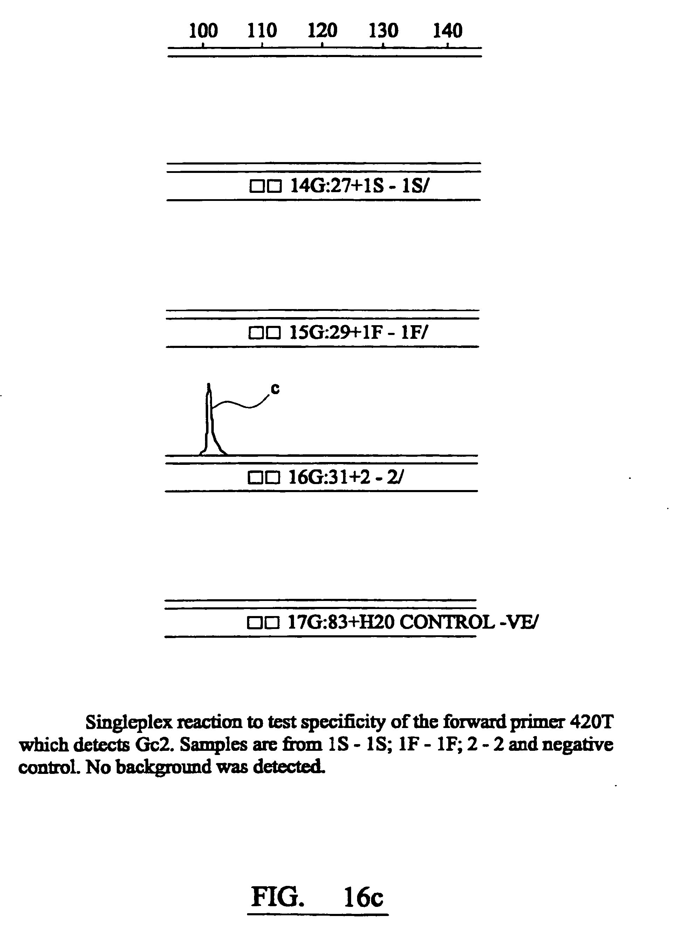

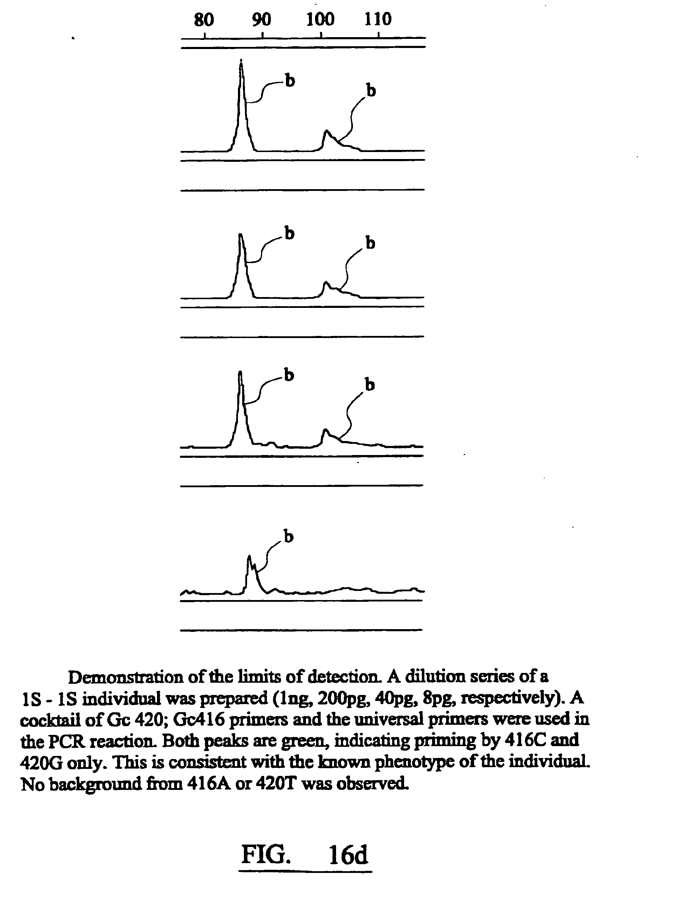

The molecular beacon approach is capable of amplifying 2ng of DNA. Using exactly the same approach, an experiment was performed to gain a measure of the sensitivity of the method. Various dilutions of a Gc1s + ve DNA sample were made and amplified as described in the previous example. The results are shown in Figure 16 which again illustrates a graph of fluorescent verses 2^{nd} round amplification cycle number for various concentrations of Gc1s + ve DNA when compared with a control, SDW.

Whilst there is some degree of non-specific fluorescence seen in the SDW control, this is clearly distinguishable even from the 0.02ng Gc1s DNA + ve and therefore has no effect with respect to interpretation of samples that contain even substantially sub-nanogram quantities of DNA. Very low levels of sample can thus be successfully analysed using this technique.

### Example C

As demonstrated above the universal G beacon can clearly identify and amplify DNA that is primarily amplified using the Gc1s primer. To demonstrate its truly universal application, both universal G and universal C beacons are demonstrated in this example as being able to amplify other first round products derived from different 1^{st} round amplification primers. To this end, 1^{st} round amplifications were performed using Gc1, Gc2, Gc1s and Gc1f primers as specified below and the 2^{nd} round amplification was carried out using universal G or C beacon together with universal 11 reverse. The 2^{nd} round amplification cycle numbers were reduced to 20 in order to minimise overamplification.

### 1^{st} round primers

| **Primer name** | **Primer sequence** |
|---|---|
| Gc1 uni 9 G | CGACGTGGTGGATGTGCTAGACCAGCTTTGCCAGTTCCG |
| Gc2 uni 9 C | CGACGTGGTGGATGTGCTTCACCAGCTTTGCCAGTTCCT |
| Gc1s uni 9 G | CGACGTGGTGGATGTGCTAGGTTCCGTGGGTGTGGCC |
| Gc1f uni 9 C | CGACGTGGTGGATGTGCTTCGTTCCGTGGGTGTGGCA |
| Gc reverse uni 11 | TGACGTGGCTGACCTGAGACGGCAGAGCGACTAAAAGCAAA |

### 2^{nd} round primers

| **Primer name** | **Primer sequence** |
|---|---|
| Universal G beacon | F-ACGCGCTCTCTTCTTCTTTTGCGCG-Q-CGACGTGGTGGATGTGCTAG |
| Universal C beacon | F-ACGCGCTCTCTTCTTCTTTTGCGCG-Q-CGACGTGGTGGATGTGCTTC |
| Universal 11 | TGACGTGGCTGACCTGAGAC |

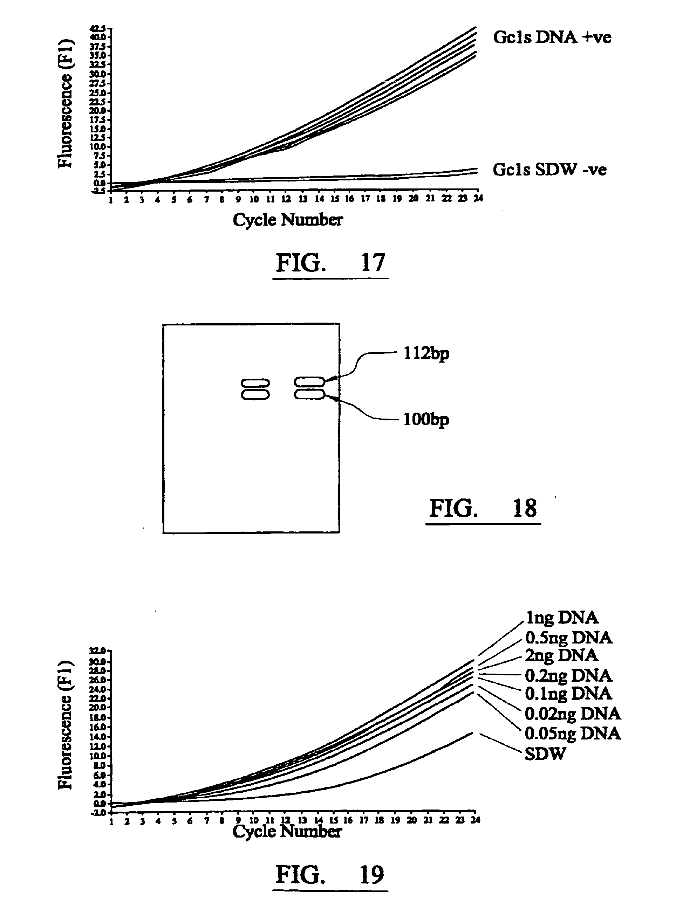

The results from this example are displayed in Figures 17a, 17b, 17c and 17d. The results from 17a and 17b show sample 1 to have a Gc 1 polymorphism and sample 2 to have a Gc2 polymorphism. Example 3 is an SDW control which produces a negative result throughout. There are two known alleles associated with the Gc1 polymorphism, designated Gc1s and Gc1f and an individual can be either homozygote for Gc1s or Gc1f, or heterozygote for Gc1s1f. As shown by Figures 17c and 17d, sample 1 amplifies in both instances and therefore indicates a Gc1s1f phenotype. As sample 2 does not amplify with Gc1s primer, this confirms its assignment as Gc2 phenotype. The amplification of sample 2 with Gc1f primers is due to the specific binding site base sequence of the Gc region. An individual who is Gc1, has a C base position 2 of codon 420, whilst a Gc2 individual has an A base at the same position. Gc 1 individuals are differentiated into Gc 1 s and Gc 1 f phenotypes characterised by the base core at position 3 of codon 416, being a G or T respectively. Individuals who are Gc2 only have a T base at position 3 in codon 416 and therefore the Gc 1f primer is able to bind complimentary at this position despite having a base mismatch with codon 420. Such occurrences result in non-specific amplification as illustrated in more detail in Figure 18.

### Example D

Further investigation were carried out on another locus, namely Amelogenin. The 1^{st} round primers are as follows:-

### 1^{st} round primers

| **Primer name** | **Primer sequence** |
|---|---|
| Amelo X | CGACGTGGTGGATGTGCTTCTGAGCCAATGGTAAACCTGCC |
| Amelo Y | CGACGTGGTGGATGTGCTAGTGAGCCAATGGTAAACCTGCA |
| Amelo reverse | TGACGTGGCTGACCTGAGACCATAGGAAGXGTACTGGTGAGAAACA |

### 2^{nd} round primers

### Primer name Primer sequence

### 2^{nd} round primers

| **Primer name** | **Primer sequence** |
|---|---|
| Universal G beacon | F-ACGCGCTCTCTTCTTCTTTTGCGCG-Q-CGACGTGGTGGATGTGCTAG |
| Universal C beacon | F-ACGCGCTCTCTTCTTCTTTTGCGCG-Q-CGACGTGGTGGATGTGCTTC |
| Universal 11 | TGACGTGGCTGACCTGAGAC |

Amplification conditions are the same as before. A male sample was amplified with the Amelo Y primer and detected using universal G beacon. The results are illustrated in Figure 19.

Once again, product was run on a 3% Nusieve minigel to confirm that the amplified product was the correct size. As seen before there were two bands present that differed by 12bp. The longer of the two bands confirmed that the amplified product was the correct size and thus proved that the additional band was likely to be the result of intramolecular modification as previously outlined. The presence of this phenomenon with two different loci implies that it is a feature of the universal molecular beacon primer structure rather than artifact derived from mispriming.

Clearly, the universal reporter primer principle (URP), is able to amplify multiple alleles within a given locus to facilitate accurate genotyping. Furthermore, they enable multiple loci to be determined making them truly universal.

### Example E

Whilst the techniques sensitivity has been demonstrated with respect to being able to amplify and detect sub-nanogram levels of DNA, this examples takes the demonstration one step further in the context of sensitivity and specificity of the technique with respect to mixtures.

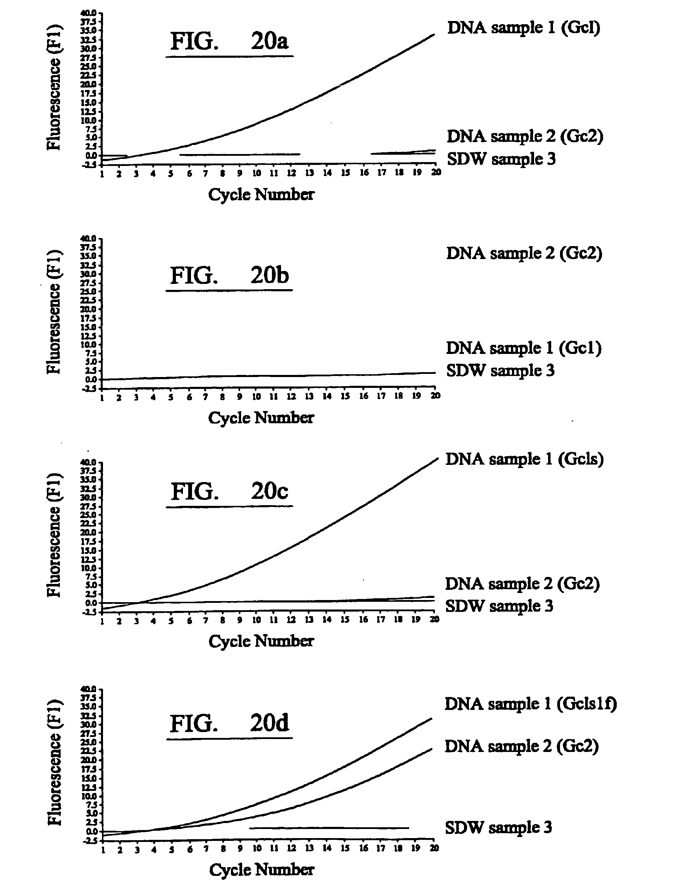

In the following example, 2 DNA's were mixed together in ratios of 1:2, 1:4, 1:6, 1:8, 1:16, 1:50, 1:100 and 1:300. In each case, the total amount of DNA present was 2ng. The mixtures were prepared with the major component coding for the Gc2 polymorphism and the minor component coding for the Gc1s polymorphism. Amplified with forward primers, either Gc2 or Gc1s respectively and a reverse Gc primer, the cycling condition were as described earlier. In the 2^{nd} round, 1^{st} round product was amplified with either universal C beacon or universal G beacon respectively, together with universal 11. Cycling conditions were as before however, the actual number of cycles for the 2^{nd} round was reduced to 12. the results are shown in the graphs of Figures 20a and 20b.

In Figure 20a all sample have amplified with the exception of the male -ve control and SDW. All other sample have amplified at the same time and rate. Slight differences between samples with respect to their absolute fluorescence is attributed to the relative increase in major component as the ratios shift from 1:2 to 1:300, (minor component to major component respectively).

Figure 20b shows that as the ratio of minor component decreases, the time taken for these sample to begin amplification also decreases, as does the rate of amplification. At 1:300, the amount of starting material is approximately 6pg but the increase in fluorescence attributed to DNA amplification in this mixture is clearly defined above baseline.

As is demonstrated we can successfully discriminate and amplify both the major component and minor component in mixed DNA source samples successfully over a wide range of minor to major ratios.

These results thus demonstrate the universal reporter primer principle and the following advantages.
a) The universal molecular beacons like the universal primers do not prime human DNA.
b) A two stage reaction or branched PCR may be the method of choice and this improves specificity.
c) Mispriming is negligible, helped by the branched PCR approach and this virtually eliminates background noise.
d) The ability of the universal beacons to amplify multiple loci in the second PCR means that only this one primer set is required. This has cost saving implications.
e) The universal molecule beacons facilitate real time detection of PCR product without the need for additional post PCR elucidative techniques ie. polyacrylamide gel electrophoresis (PAGE).
f) There is a distinct advantage with using this technique when sample quantity is limited, (a problem commonly encountered in forensic biology when sub-nanogram levels of material may be recovered). 1^{st} round PCR produces significant product. Aliquots of this product can then be used for several subsequent second stage PCR reactions.
g) The universal beacons are extremely sensitive with respect to mixtures and can accurately determine a minor component equal to 1:300 dilution (6pg).

### EXAMPLE - Distinct universal primer portions, attachments to glass slides and hybridisation

As described above, particularly powerful tests can be obtained if the first set of primers include within their universal primer portions, distinguishing portions, with the subsequent amplification primers being attached to glass slides and then subjected to hybridisation based analysis.

More details of the experimental method for such a system follow.

### PCR CONDITIONS

### For multiplex reactions

| | |
|---|---|
| Locus specific primer concentrations | 10nM-40nM |
| Universal reporter primer concentrations | 100-1000nM |
| Mg-ion concentration | 1.5nM |
| Buffer | Perkin Elmer PCR buffer X 1 |
| | concentration |
| DNA | 1nG |
| Reaction volume | 50uL |
| dNTP's | 200uM |
| Taq gold | 1.25U/50uL |

Cycling conditions:
95 °C for 10 minutes - Taq activation
94°C for 30 seconds
60°C for 30 seconds
72 °C for 90 seconds
performed for 32 to 40 cycles.

### Hybridisation conditions

Hybridisation was performed at a temperature of 40°C to 60°C to promote hybridisation of the probes. The hybridisation solution consists of a solution containing - 0.5M di sodium hydrogen orthophosphate, pH7.2, 1mM EDTA and 7% SDS. Hybridisation probes were dissolved in the solution at a concentration of 0.1-5uM. A volume of 80-120ul of hybridisation solution was applied to the slide which was then covered with a cover slip, placed in a humid atmosphere at 40°C to 60°C for two hours.

After hybridisation the slides were washed in the solution of 0.1 to 4 x 55C and 0.1%SDS at 60°C t 75°C.

When dry, the slides are scanned with a molecular dynamics erase scanner in order to detect the fluorescent probe.

### Multiplex analysis

Such systems are suited for use in multiplex conditions. The amplified products are spotted on to a slide as previously described, and each spot is then visualised for specific loci using successive hybridisations. Other loci present within the amplification products will not hybridise and hence will not contribute to the detected results during each of the respective hybridisations and investigations. After the determination of a particular locus, slides can be immersed in an appropriate solution to remove all of the hybridisation probes found to the target DNA present from that hybridisation. The covalent bonding of the strands means that they are maintained on the slide.

Suitable controls can be used to demonstrate that DNA is actually present in each spot. Controls comprising a mixture of oligonucleotides that are complimentary to each universal primer portion utilised (omitting the specific primer region) are envisaged. The slide would hybridise with the control probes, and the signal would be proportional to the amount of DNA actually present in the spot.

To obtain the full benefits of the present invention's technique it is desirable to deploy it in multiplex reactions so that a large number of loci can be considered simultaneously. A problem with multiplex reactions in general is that it is difficult to prevent significant primer dimer formation as different primers are used, and as the concentration of primers increases within the reaction mix. Once a primer dimer forms the PCR reaction tends to produce primer dimer preferentially and hence the reaction becomes very inefficient. Primer dimer forms arise when primers are themselves complimentary to other primers in the multiplex reaction mix.

Two techniques to address this issue have been developed by the applicants and those will now be described.

In the first technique, locus specific primers are used in a first set of primers and universal primers are used in a second set. Both sets are incorporated into a single reaction, however. The second set of primers are provided at a far higher concentration, however, than the locus specific primers of the first set. Concentrations of 400nM-4000nM compared with 10-200nM are deployed in the reaction mix. The universal primers of the second set employ dye labels as previously mentioned.

The key to avoiding primer dimer formation is the temperature employed in the PCR reaction during the various cycles.

During the first two cycles of PCR amplification only the locus specific portion of the locus specific universal primers initiate priming. Once the complimentary sequence to the universal portion has formed as a result of the first two cycles in subsequent rounds of amplification the entire first stage primer is able to prime as now both the locus specific and universal primer portions of the primer are present in the PCR product.

Increasing the annealing phase temperature to 72°C to 76°C during the early amplification cycles, for instance cycles 4 to 24, means that priming due to the first stage primer occurs but that annealing of the second stage primer, the universal primer carrying the dye label, is inhibited entirely. The annealing temperature is simply too high for the second stage primer to anneal. Once sufficient amplification has occurred, the annealing temperature for subsequent stages is reduced to around 60°C and this allows the universal primers to prime. As a consequence subsequent amplification cycles result in priming of this second stage primer also and hence in the incorporation of the fluorescent dye labels into the amplification products. Usually only a couple of cycles are needed to effect this part of the process.

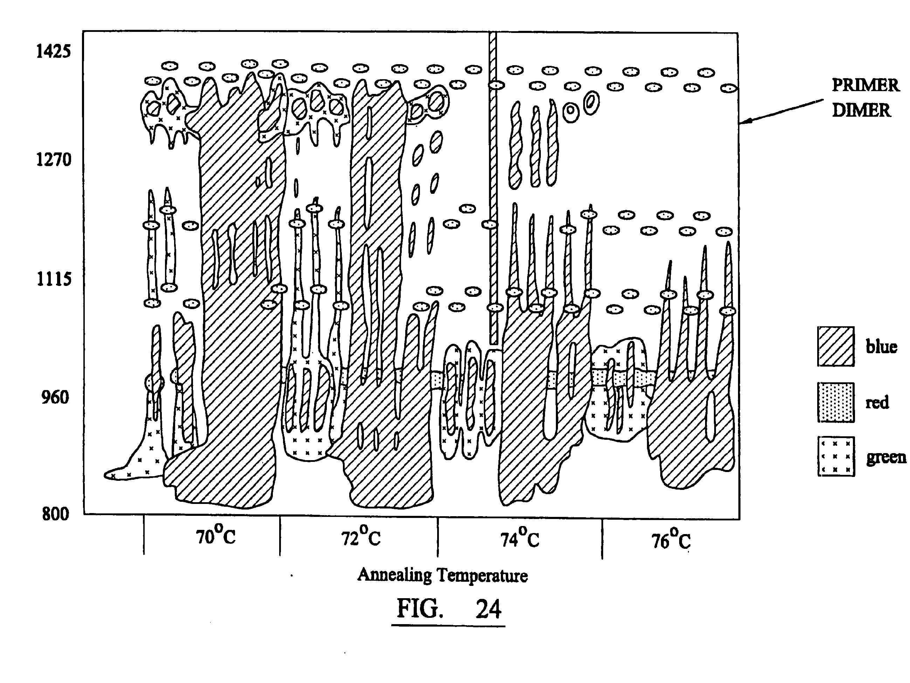

The overall result of the use of low concentrations and the first primer stage at high annealing temperatures, with the universal primers switched off as a result is that primer dimer formation is minimised. The impact of careful temperature control during annealing on the process is highlighted in Figure 24 where at 70 and 72°C for the annealing temperature very substantial primer dimer formation occurs. By 74°C for the annealing temperature, this is decreased very substantially, and at 76°C is almost eliminated.

The appropriate annealing temperature to obtain such a benefit for a primer can be established through a series of experiments at different annealing temperatures.

Providing the amplification process in this way, with relatively low concentrations of the first stage primers, is also beneficial in addressing the variation in efficiencies of amplification which exist between primers. Starting from low concentrations means that the most efficient primers will be rapidly depleted as the reactions progress whereas the less efficient primers will be less rapidly depleted. Over a suitable number of reaction cycles this allows the less efficient primers to catch up and provide generally equivalent levels of amplification.

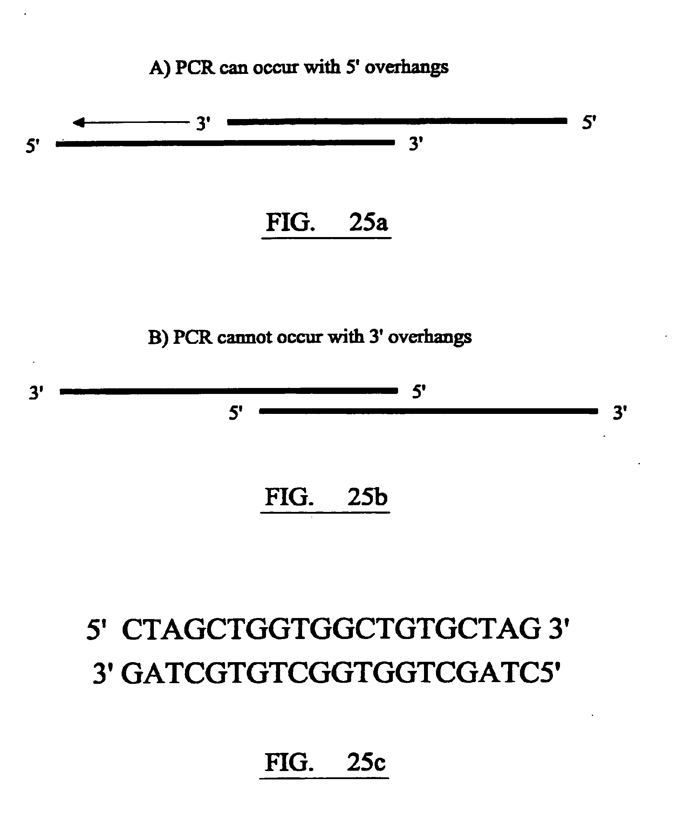

As an alternative or additional technique to assist in the avoidance of primer dimer formation, it is possible to carefully design the second stage universal primers. Primer dimer formation occurs if two primers can hybridise with one another such that the 5' ends overhang, thereby allowing extension from the 3' end, Figure 25a. Primer dimer formation cannot occur from 3' end overhangs, Figure 25b. As a consequence the present invention aims to ensure that the primers can only possibly hybridise with one another to give 3' end overhangs.

The design process for the universal primers involves the provision of self complimentary 3' end and 5' end sequences. As shown in Figure 25c, two universal primers are provided to address a biallelic situation which is being analysed. If all four possibilities for the SNP apply, then four similarly provided universal primers can be employed.

The primers consist of the two complimentary end portions and an allele specific sequence provided therebetween. If desired for use as the first stage primer, a locus specific primer portion can be attached to either 3' end of these primers. Such sequences are illustrated in Figure 11 above and this is the reason why the discriminating portion of the further portion is an intervening sequence rather than provided at the end.

### Examples - Absence of Second Stage Reverse Primer

As a refinement of some of the examples discussed above, analysis using the same primers as described on page 38 above was performed, but without the universal 11 reverse primer.

The PCR conditions and cycles are as follows:-

| **Primer** | **Optimal Concentration** | |
|---|---|---|
| | **Beacon Assay** | **Sybr Green Assay** |
| 1^{st} round forward primer | 40nM | 40nM |
| 1^{st} round reverse primer | 100nM | 100nM |
| 2^{nd} round forward primer | 200nM | - |
| 2^{nd} round reverse primer | - | - (OMITTED IN THIS METHOD) |
| Sybr Green | - | 10% |
| MgCl₂ | 1.5mM | 1.5mM |
| PE Buffer | 10% | 10% |
| d NTP's | 200µM | 200µM |
| Taq Gold | 0.1 U/reaction | 0.1 U/reaction |
| DNA | 2ng/reaction | 2ng/reaction |
| SDW | | |

### Reaction Volume = 20µl

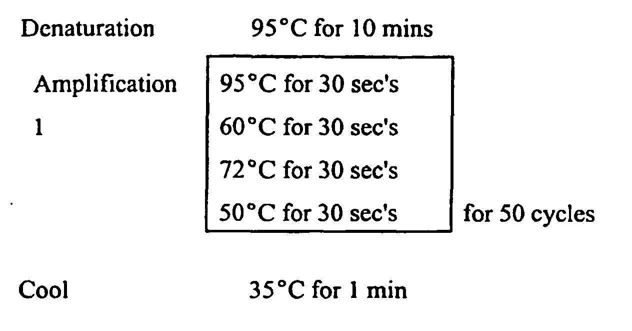

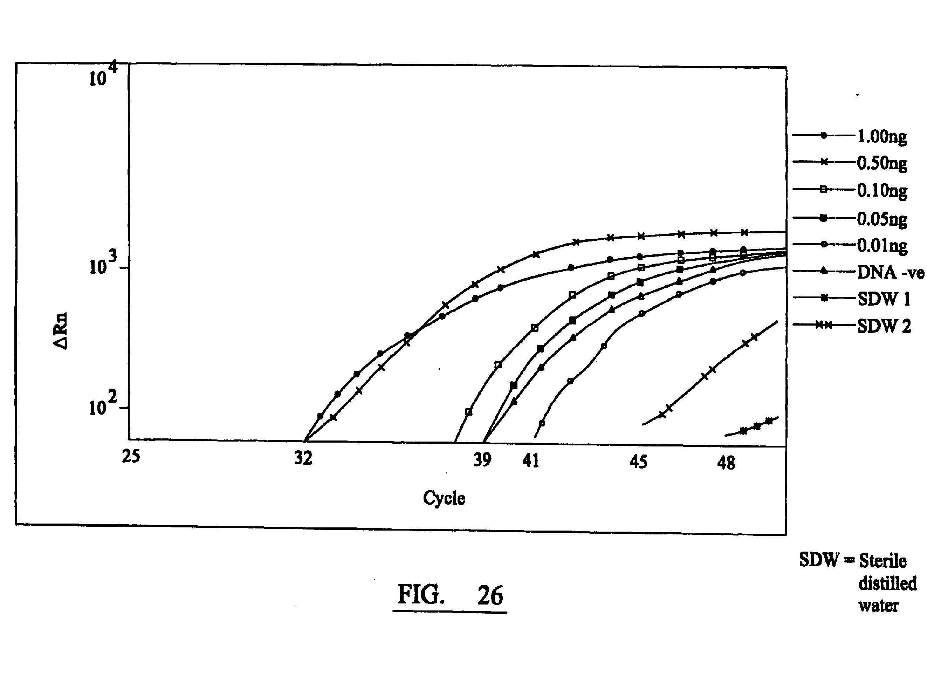

In this example, the following amounts of DNA, (Ing, 0.5ng, 0.05ng and 0.01ng), were amplified with the redesigned Gc1s primers in the presence of the molecular beacon assay, a graph showing fluorescence against cycle number for the various sample sizes amplified is illustrated in Figure 26

The beacons that incorporate universal 9 have provided a much better result under the revised amplification protocol. All products are sizing at approximately 112bp and there is no detectable presence of primer dimer.

The Ct value is defined as the cycle number at which the reporter fluorescence increases above a base line or threshold level. This level is usually set at a position directly at the start of exponential amplification when PCR amplification is optimised. This assay can unambiguously differentiate <0.01ng (10pg) genomic DNA from SDW. However, note that the DNA negative control comprises 2ng of a Gc2 individual. This is in great excess compared to the quantities of DNA analysed. With this amount of DNA a small amount of mis-priming will inevitably occur. 2ng of Gc2 gives an apparent ct that is equivalent to c.50pg (which is only 1/40th the actual amount). In real casework it is very unlikely that such large quantities of DNA will be used - this means that the test is highly allele specific. Note that the sterile distilled water negatives are completely clean.

### SYBR GREEN ASSAY

The same primers were used as described for the molecular beacon assay except that the molecular beacon itself is not used. All other protocols are described were described in the previous section.

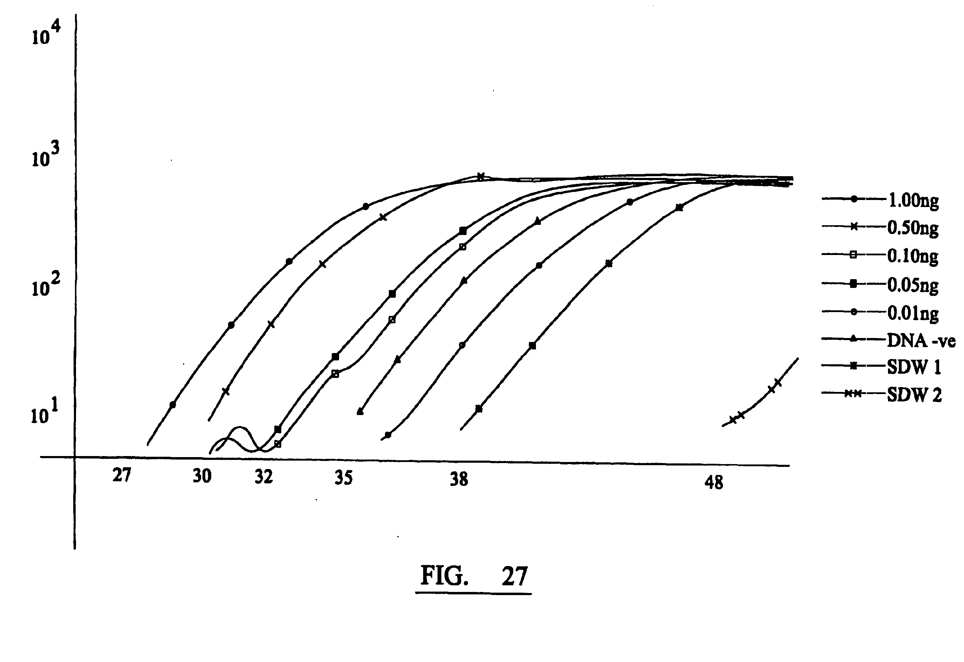

The results in Figure 27 show very similar sensitivity to that previously described for the molecular beacon experiment previously described.

### DEMONSTRATION OF NON-5' END OVERHANG PRIMERS FOR SYBR GREEN ASSAY

The same protocols as previously described were followed - the following primers are used:-

### Primer set C

Gc 1s URP 13.1 G CTAGCTGGTGGCTGTGCTAGGTTCCGTGGGTGTGGCC
Gc reverse URP 13.1 CTAGCTGGTGGCTGTGCTAGGGCAGAGCGACTAAAAGCAAA

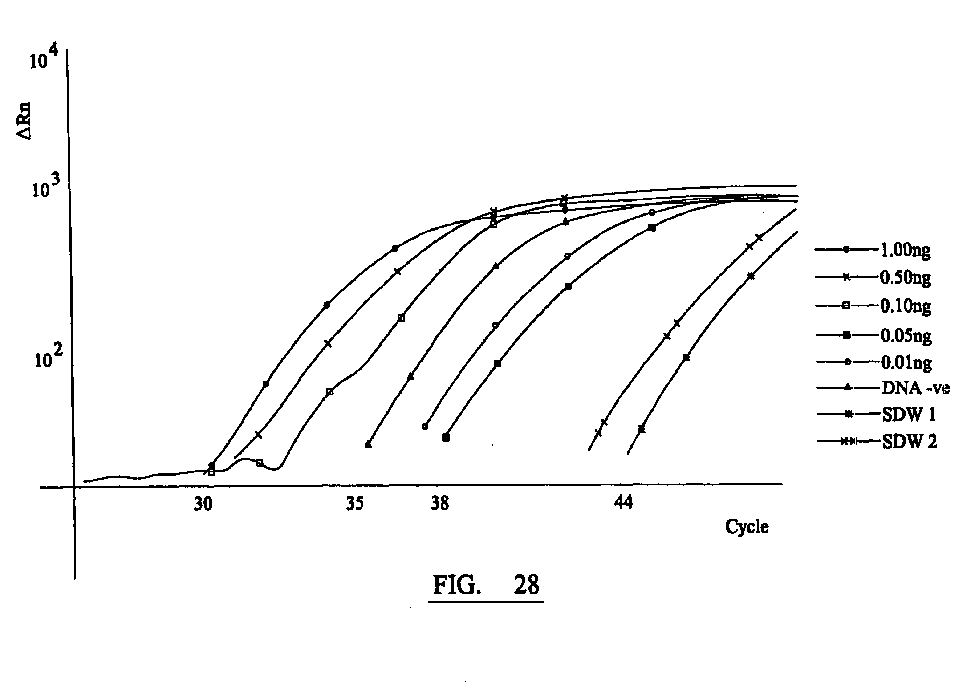

Results are shown in Figure 28. Again results are similar to those previously described and achieved good results even for very small samples.

To summarise, we demonstrate that the Sybr Green, a dye, and molecular beacon assays that utilise the principals set out above are able to analyse concentrations of DNA <100pg. Furthermore the reaction shows considerable allele specificity. A negative control that comprises a different allele (Gc2) at a very high level of 2 ng showed a small amount of mispriming with the Gc1s primer at approximately the 10pg level.

The technique of the present invention, as exemplified through its various embodiments in particular, is suitable for analysing very low levels of DNA contained in a mixture. The technique has the ability to pick out DNA from a source which is the minor contributor to a mixture by a long way. Thus, the technique enables mixtures in which one source only contributes to the sample in the ratio 1:5000 to be successfully picked out. This is useful where samples from mixed sources but one party is likely to contribute in a small amount. The technique is also applicable in enabling very small quantities of male DNA to be picked out and analysed in the presence of substantial quantities of female DNA. The sample including 0.02% male DNA, or even less, can successfully be analysed using this invention even when 99.98% of the sample is female DNA.

In any cases where low levels of sample are being analysed, the level of sample present can be considered by comparing the fluorescence level arising from the unknown sample against a series of calibration results, forming a curve, with those results being based on different levels of DNA as a portion of the overall DNA sample.

## Claims

1. A method of investigating single nucleotide polymorphisms in a sample of DNA, the method comprising
contacting the DNA containing sample with a first set of primers, the first sets of primers including two or more primers including a locus specific portion and a further portion, the further portion of at least one of the primers being different from the further portion of at least one of the other primers,
amplifying the DNA, the amplification using the first sets of primers to give an amplified product, the amplified product including a sequence complementary to the locus specific portion and further portion of a first set primer,
contacting at least a portion of the amplified product with at least one second set of primers,
amplifying the first amplified product to give a further amplified product by hybridising or annealing at least one of the second set of primers to that part of the first amplified product with a sequence complementary to the further portion and
examining one or more characteristics of the further amplified product using the presence or absence of a distinctive unit introduced by hybridisation or annealing of a component, which includes the distinctive unit, to the sequence complimentary to the further portion.

2. A method according to claim 1 in which two or more of the primers of the second set of primers including a second further portion, the second further portion of one of the primers including a sequence which matches the sequence of at least part of the further portion of one of the primers of the first set, the second further portion of one of the primers including a sequence which matches the sequence of at least part of the further portion of a different one of the primers of the first set.

3. A method according to claim 1 or claim 2 in which the distinctive unit is introduced during the amplification process.

4. A method according to claim 1 or claim 2 in which the distinctive unit is introduced in a step which is subsequent to the amplification process.

5. A method according to claim 4 in which the component is a probe for the further portion.

6. A method according to any preceding claim in which the first sets of primers consist of two forward primers and a reverse primer.

7. A method according to any preceding claim in which the further portion of the first set of primers is attached to the 5' end of the locus specific portion.

8. A method according to any preceding claim in which the 3' end of the forward primers of the first sets of primers are provided with a SNP identifying portion.

9. A method according to claim 8 in which the locus specific portion and SNP identifying portion of one of the forward primers anneals to the 3' side of the locus having the SNP under investigation and the locus specific portion and SNP identifying portion of the other of the forward primers of that first set does not anneal to the 3' side of the SNP under investigation.

10. A method according to claim 8 in which the annealing primer anneals due to a match between the SNP identifying portion and the SNP site of the sample and the non-annealing primer does not anneal due to a mis-match between the SNP identifying portion and the SNP site of the sample.

11. A method according to any preceding claim in which only one second set of primers is provided.

12. A method according to any preceding claim in which the second set of primers consists of two forward primers and a reverse primer.

13. A method according to any preceding claim in which the second further portion includes a sequence which matches the sequence of the first further portion and / or pairs to the sequence of the amplified product matching the first further portion.

14. A method according to any preceding claim in which a plurality of first sets of primers are provided to amplify a plurality of SNP loci, the amplification products resulting being of different lengths.

15. A method according to any preceding claims in which a first set of amplifying conditions and a second set of amplifying conditions are employed, the first set of amplifying conditions inhibiting amplification by one or more of the primers.

16. A method according to claim 15 in which the second set of amplifying conditions provides for amplification of the previously inhibited one or more primers.

17. A method according to any preceding claim in which the first and second set of primers are present together and the annealing temperature for at least some of the cycles of the amplification process is such that at least 80% of the second set of primers remain single stranded.

18. A method according to claim 17 in which the annealing temperature is so provided and used at least in cycles 3 to 30.

19. A method according to any preceding claim in which an annealing temperature is used in at least the last two cycles, the annealing temperature allowing at least 80% of the second set of primers to anneal.

20. A method according to any preceding claim in which the annealing temperature is at least 72°C for cycles 3 to 30 of the amplification process.

21. A method according to any preceding claim in which the annealing temperature for at least the last two cycles of the amplification process is 62°C or less.

22. A method according to any preceding claim in which the first and second set of primers are present together and in which the concentration of the second set of primers is provided in a ratio relative to the concentration of the first set of primers of at least 5:1.

23. A method according to any preceding claim in which the first and second set of primers are present together, the first set of primers is provided at a concentration of between 10 and 200nM and the second set is provided at a concentration of between 400 and 4000nM.

24. A method according to any preceding claim in which the distinctive unit is a dye, dye label, colour producing molecule, molecular beacon, emitter of radiation, characteristic isotope.

25. A method according to claim 3 or any claim depending thereon in which a distinctive unit is introduced during amplification.

26. A method according to claim 3 or any claim depending thereon in which a distinctive unit is provided at the 5' end of the forward primers of the second set of primers, a different distinctive unit being provided for each forward primer in a second set.

27. A method according to any preceding claim in which the distinctive unit is indicative of the nucleotide presence at the SNP.

28. A method according to any preceding claim in which the amplification products of two or more first sets of primers and one or more second sets of primers are separated from one another using electrophoresis.

29. A method according to claim 4 or any claim depending thereon in which the further amplified product includes an attachment unit and the attachment unit facilitates attachment of the further amplified product to a solid support.

30. A method according to claim 29 in which the attached further amplified product is contacted with one or more probes having different sequences from one another, at least in part, the contact resulting in hybridisation of one of the probes to the further amplified product.

31. A method according to claim 29 or claim 30 in which each probe has a common sequence portion to each other, the common sequence portion corresponding in sequence to the locus specific portion of the further amplified product.

32. A method according to any of claims 29 to 31 in which the probes incorporate at least one different sequence portion compared with one another, the different sequence portion of at least one of the probes corresponding to the sequence of the further portion which is included in the sequence of the further amplified product.

33. A method according to any of claims 29 to 32 in which contact of the probes with the further amplified product results in hybridisation of one of the probes to the further amplified product, each probe having a distinctive unit relative to one another.

34. A method according to any preceding claim in which one or more of the first set of primers are provided with an SNP identifying portion, the SNP identifying portion being different for each different primer, the primer with an SNP identity portion which pairs to the SNP, annealing one side of the SNP.

35. A method according to any preceding claim in which the locus specific portion of the primers of the first set includes a sequence which matches the sequence of the locus sequence in the vicinity of the SNP under investigation, the match between the locus specific portion and sequence of the locus commencing at between one and ten bases to the respective sides of the SNP under investigation.

36. A method according to any preceding claim in which the first set of primers includes a reverse primer and further includes a forward primer for each possible identity of the SNP under investigation.

37. A method according to any preceding claim in which the locus specific portion of the primers in a set are provided with identical sequences in each primer.

38. A method according to any preceding claim in which the further portion includes a sequence which does not match the locus sequence on the 3' side of the locus with which the locus specific portion of the primer matches.

39. A method according to any preceding claim in which the further amplified product is contacted with one or more components retained on a solid support, the one or more components having a sequence which anneals with at least part of the sequence of one of the further amplified products.

40. A method according to claim 39 in which the retained component anneals with the further amplified product up to the base before the base which is the SNP side.

41. A method according to claim 39 in which the retained component anneals to the further amplified product along the sequence corresponding to the locus specific portion and further portion of the further amplified product.

42. A method according to any of claims 39 to 41 in which a plurality of different retained components, preferably PCR products and / or oligonucleotides, are provided at discrete locations on a support, different retained components annealing to different further amplified products.

43. A method according to claim 41 in which the retained component and annealed further amplified product are contacted with one or more further components to introduce a distinctive unit.

44. A method according to claim 41 in which the retained component and annealed further amplified product are contacted with one or more additional components, the one or more additional component being one or more further oligonucleotides which include a distinctive unit.

45. A method according to claim 44 in which the end base of the further oligonucleotide is one of the four possible identities for the SNP.

## Patentansprüche

1. Verfahren zur Untersuchung von Einzelnucleotidpolymorphismen in einer DNA-Probe, wobei das Verfahren umfasst:
In-Kontakt-Bringen der DNA-enthaltenden Probe mit einem ersten Primersatz, wobei die ersten Primersätze zwei oder mehr Primer, die einen lokusspezifischen Teil und einen weiteren Teil einschließen, wobei der weitere Teil mindestens eines der Primer unterschiedlich zu dem weiteren Teil mindestens eines der anderen Primer ist, enthalten,
Amplifikation der DNA, wobei die Amplifikation die ersten Primersätze verwendet, um ein amplifiziertes Produkt zu liefern, wobei das amplifizierte Produkt eine zu dem lokusspezifischen Teil und weiteren Teil eines ersten Primersatzes komplementäre Sequenz einschließt,
In-Kontakt-Bringen von mindestens einem Teil des amplifizierten Produkts mit mindestens einem zweiten Primersatz,
Amplifikation des ersten amplifizierten Produkts, um ein weiter amplifiziertes Produkt durch Hybridisierung oder Anlagerung mindestens eines aus dem zweiten Primersatz an den Teil des ersten amplifizierten Produkts mit einer zu dem weiteren Teil komplementären Sequenz zu liefern, und
Untersuchung einer oder mehrerer Eigenschaften des weiter amplifizierten Produkts durch Verwendung der Anwesenheit oder Abwesenheit einer Unterscheidungseinheit, die durch Hybridisierung oder Anlagerung eines Bestandteils, der die Unterscheidungseinheit enthält, in die zu dem weiteren Teil komplementäre Sequenz eingeführt wird.

2. Verfahren gemäß Anspruch 1, in dem zwei oder mehr der Primer des zweiten Primersatzes einen zweiten weiteren Teil einschließen, wobei der zweite weitere Teil eines der Primer eine Sequenz einschließt, die zu den Sequenzen mindestens eines Teils des weiteren Teils eines der Primer des ersten Satzes passt, der zweite weitere Teil eines der Primer eine Sequenz einschließt, die zu der Sequenz mindestens eines Teils des weiteren Teils eines unterschiedlichen Primers der Primer des ersten Satzes passt.

3. Verfahren gemäß Anspruch 1 oder Anspruch 2, in dem die Unterscheidungseinheit während des Amplifikationsprozesses eingeführt wird.

4. Verfahren gemäß Anspruch 1 oder Anspruch 2, in dem die Unterscheidungseinheit in einem Schritt, der im Anschluss an den Amplifikationsprozess ist, eingeführt wird.

5. Verfahren gemäß Anspruch 4, in dem der Bestandteil eine Sonde für den weiteren Teil ist.

6. Verfahren gemäß einem der vorangehenden Ansprüche, in dem die ersten Primersätze aus zwei Forward-Primern und einem Reverse-Primer bestehen.

7. Verfahren gemäß einem der vorangehenden Ansprüche, in dem der weitere Teil des ersten Primersatzes an das 5'-Ende des lokusspezifischen Teils gebunden ist.

8. Verfahren gemäß einem der vorangehenden Ansprüche, in dem das 3'-Ende der Forward-Primer der ersten Primersätze mit einem SNP-identifizierenden Teil versehen ist.

9. Verfahren gemäß Anspruch 8, in dem der lokusspezifische Teil und SNP-identifizierende Teil eines der Forward-Primer an die 3'-Seite des Lokus, der den zu untersuchenden SNP aufweist, anlagern, und der lokusspezifische Teil und SNP-identifizierende Teil des anderen der Forward-Primer dieses ersten Satzes nicht an die 3'-Seite des zu untersuchenden SNP anlagern.

10. Verfahren gemäß Anspruch 8, in dem sich der Anlagerungsprimer wegen einer Übereinstimmung zwischen dem SNP-identifizierenden Teil und der SNP-Stelle der Probe anlagert und sich der Nicht-Anlagerungsprimer wegen einer fehlenden Übereinstimmung zwischen dem SNP-identifizierenden Teil und der SNP-Stelle der Probe nicht anlagert.

11. Verfahren gemäß einem der vorangehenden Ansprüche, in dem nur ein zweiter Primersatz bereitgestellt wird.

12. Verfahren gemäß einem der vorangehenden Ansprüche, in dem der zweite Primersatz aus zwei Forward-Primern und einem Reverse-Primer besteht.

13. Verfahren gemäß einem der vorangehenden Ansprüche, in dem der zweite weitere Teil eine Sequenz einschließt, die zu der Sequenz des ersten weiteren Teils passt und/oder sich mit der Sequenz des amplifizierten Produkts, das zu dem ersten weiteren Teil passt, paart.

14. Verfahren gemäß einem der vorangehenden Ansprüche, in dem eine Vielzahl von ersten Primersätzen bereitgestellt wird, um eine Vielzahl von SNP-Loki zu amplifizieren, wobei die resultierenden Amplifikationsprodukte verschiedene Längen haben.

15. Verfahren gemäß einem der vorangehenden Ansprüche, in dem ein erster Satz von Amplifikationsbedingungen und ein zweiter Satz von Amplifikationsbedingungen eingesetzt werden, wobei der erste Satz der Amplifikationsbedingungen eine Amplifikation durch einen oder mehrere der Primer inhibiert.

16. Verfahren gemäß Anspruch 15, in dem der zweite Satz der Amplifikationsbedingungen für eine Amplifikation des/der zuvor inhibierten einen oder mehreren Primer sorgt.

17. Verfahren gemäß einem der vorangehenden Ansprüche, in dem der erste und zweite Primersatz zusammen vorliegen und die Anlagerungstemperatur für mindestens einige Zyklen des Amplifikationsprozesses so ist, dass mindestens 80% des zweiten Primersatzes einzelsträngig bleiben.

18. Verfahren gemäß Anspruch 17, in dem die Anlagerungstemperatur mindestens in den Zyklen 3 bis 30 so zur Verfügung gestellt und verwendet wird.

19. Verfahren gemäß einem der vorangehenden Ansprüche, in dem eine Anlagerungstemperatur in mindestens den letzten zwei Zyklen verwendet wird, wobei die Anlagerungstemperatur ermöglicht, dass sich mindestens 80% des zweiten Primersatzes anlagern.

20. Verfahren gemäß einem der vorangehenden Ansprüche, in dem die Anlagerungstemperatur mindestens 72°C für die Zyklen 3 bis 30 des Amplifikationsprozesses ist.

21. Verfahren gemäß einem der vorangehenden Ansprüche, in dem die Anlagerungstemperatur für mindestens die letzten zwei Zyklen des Amplifikationsprozesses 62°C oder niedriger ist.

22. Verfahren gemäß einem der vorangehenden Ansprüche, in dem der erste und zweite Primersatz zusammen vorliegen und in dem die Konzentration des zweiten Primersatzes in einem Verhältnis von mindestens 5:1, relativ zu der Konzentration des ersten Primersatzes, zur Verfügung gestellt wird.

23. Verfahren gemäß einem der vorangehenden Ansprüche, in dem der erste und zweite Primersatz zusammen vorliegen, der erste Primersatz in einer Konzentration zwischen 10 und 200 nM zur Verfügung gestellt wird und der zweite Satz in einer Konzentration zwischen 400 und 4000 nM zur Verfügung gestellt wird.

24. Verfahren gemäß einem der vorangehenden Ansprüche, in dem die Unterscheidungseinheit ein Farbstoff, eine Farbstoffmarkierung, ein Farbe erzeugendes Molekül, molekulares Lichtsignal, Emitter von Strahlung, charakteristisches Isotop ist.

25. Verfahren gemäß Anspruch 3 oder einem der davon abhängigen Ansprüche, in dem eine Unterscheidungseinheit während der Amplifikation eingeführt wird.

26. Verfahren gemäß Anspruch 3 oder einem der davon abhängigen Ansprüche, in dem eine Unterscheidungseinheit an dem 5'-Ende der Forward-Primer des zweiten Primersatzes zur Verfügung gestellt wird, wobei eine andere Unterscheidungseinheit für jeden Forward-Primer in einem zweiten Satz zur Verfügung gestellt wird.

27. Verfahren gemäß einem der vorangehenden Ansprüche, in dem die Unterscheidungseinheit eine Nucleotidpräsenz bei dem SNP aufzeigt.

28. Verfahren gemäß einem der vorangehenden Ansprüche, in dem die Amplifikationsprodukte der zwei oder mehr ersten Primersätze und die der ein oder mehr zweiten Primersätze durch Verwendung einer Elektrophorese von einander getrennt werden.

29. Verfahren gemäß Anspruch 4 oder einem der davon abhängigen Ansprüche, in dem das weiter amplifizierte Produkt eine Bindungseinheit einschließt und die Bindungseinheit eine Bindung des weiter amplifizierten Produkts an einen festen Träger erleichtert.

30. Verfahren gemäß Anspruch 29, in dem das gebundene weiter amplifizierte Produkt mit einer oder mehr Sonden, die zumindest zum Teil voneinander verschiedene Sequenzen haben, in Kontakt gebracht wird, wobei der Kontakt in einer Hybridisierung einer der Sonden mit dem weiter amplifizierten Produkt resultiert.

31. Verfahren gemäß Anspruch 29 oder Anspruch 30, in dem jede Sonde einen gemeinsamen Sequenzteil hat, wobei der gemeinsame Sequenzteil in der Sequenz dem lokusspezifischen Teil des weiter amplifizierten Produkts entspricht.

32. Verfahren gemäß einem der Ansprüche 29 bis 31, in dem die Sonden mindestens einen verschiedenen Sequenzteil verglichen mit einer anderen einschließen, wobei der verschiedene Sequenzteil mindestens einer der Sonden der Sequenz des weiteren Teils, der in die Sequenz des weiter amplifizierten Produkts eingeschlossen ist, entspricht.

33. Verfahren gemäß einem der Ansprüche 29 bis 32, in dem ein Kontakt der Sonden mit dem weiter amplifizierten Produkt in einer Hybridisierung einer der Sonden mit dem weiter amplifizierten Produkt resultiert, wobei jede Sonde in Bezug zu einer anderen eine Unterscheidungseinheit hat.

34. Verfahren gemäß einem der vorangehenden Ansprüche, in dem einer oder mehrere aus dem ersten Primersatz mit einem SNP-identifizierenden Teil versehen sind, wobei der SNP-identifizierende Teil für jeden verschiedenen Primer verschieden ist, wobei sich der Primer mit einem SNP-Identitätsteil, der sich mit dem SNP paart, an eine Seite des SNP anlagert.

35. Verfahren gemäß einem der vorangehenden Ansprüche, in dem der lokusspezifische Teil der Primer des ersten Satzes eine Sequenz einschließt, die zu der Sequenz der Lokussequenz in der Nachbarschaft des zu untersuchenden SNP passt, wobei die Übereinstimmung zwischen dem lokusspezifischen Teil und einer Sequenz des Lokus bei zwischen einer und zehn Basen an der jeweiligen Seite des zu untersuchenden SNP beginnt.

36. Verfahren gemäß einem der vorangehenden Ansprüche, in dem der erste Primersatz einen Reverse-Primer einschließt und weiter einen Forward-Primer für jede mögliche Identität des zu untersuchenden SNP einschließt.

37. Verfahren gemäß einem der vorangehenden Ansprüche, in dem der lokusspezifische Teil der Primer in einem Satz mit identischen Sequenzen in jedem Primer zur Verfügung gestellt wird.

38. Verfahren gemäß einem der vorangehenden Ansprüche, in dem der weitere Teil eine Sequenz einschließt, die nicht zu der Lokussequenz an der 3'-Seite des Lokus, zu der der lokusspezifische Teil des Primers passt, passt.

39. Verfahren gemäß einem der vorangehenden Ansprüche, in dem das weiter amplifizierte Produkt mit einem oder mehreren Bestandteilen, die auf einem festen Träger gehalten werden, in Kontakt gebracht wird, wobei der eine oder die mehreren Bestandteile eine Sequenz haben, die sich an mindestens einem Teil der Sequenz eines der weiter amplifizierten Produkte anlagert.

40. Verfahren gemäß Anspruch 39, in dem sich der gehaltene Bestandteil an das weiter amplifizierte Produkt bis zu der Base vor der Base, die die SNP-Seite ist, anlagert.

41. Verfahren gemäß Anspruch 39, in dem sich der gehaltene Bestandteil an das weiter amplifizierte Produkt entlang der Sequenz, entsprechend dem lokusspezifischen Teil und einem weiteren Teil des weiter amplifizierten Produkts, anlagert.

42. Verfahren gemäß einem der Ansprüche 39 bis 41, in dem eine Mehrzahl verschiedener gehaltener Bestandteile, bevorzugt PCR-Produkte und/oder Oligonucleotide, an diskreten Orten auf einem Träger zur Verfügung gestellt werden, wobei sich verschiedene gehaltene Bestandteile an verschiedene weiter amplifizierte Produkte anlagern.

43. Verfahren gemäß Anspruch 41, in dem der gehaltene Bestandteil und ein angelagertes weiter amplifiziertes Produkt mit einem oder mehreren weiteren Bestandteilen in Kontakt gebracht werden, um eine Unterscheidungseinheit einzuführen.

44. Verfahren gemäß Anspruch 41, in dem der gehaltene Bestandteil und ein angelagertes weiter amplifiziertes Produkt mit einem oder mehreren zusätzlichen Bestandteilen in Kontakt gebracht wird, wobei der eine oder die mehreren zusätzlichen Bestandteile ein oder mehrere weitere Oligonucleotide, die eine Unterscheidungseinheit einschließen, sind.

45. Verfahren gemäß Anspruch 44, in dem die Endbase des weiteren Oligonucleotids eine der vier möglichen Identitäten für den SNP ist.

## Revendications

1. Procédé permettant d'étudier des polymorphismes mononucléotidiques dans un échantillon d'ADN, le procédé comprenant
de mettre en contact l'échantillon contenant de l'ADN avec un premier ensemble d'amorces, les premiers ensembles d'amorces comprenant deux amorces ou plus comprenant une partie spécifique d'un locus et une autre partie, l'autre partie d'une des amorces au moins étant différente de l'autre partie d'une des autres amorces au moins,
d'amplifier l'ADN, l'amplification utilisant les premiers ensembles d'amorces pour donner un produit amplifié, le produit amplifié comprenant une séquence complémentaire à la partie spécifique d'un locus et à l'autre partie d'une amorce d'un premier ensemble,
de mettre en contact au moins une partie du produit amplifié avec au moins un second ensemble d'amorces,
d'amplifier le premier produit amplifié pour donner un produit davantage amplifié par hybridation ou appariement d'au moins une amorce du second ensemble d'amorces avec la partie du premier produit amplifié ayant une séquence complémentaire à l'autre partie, et
d'examiner une ou plusieurs caractéristiques du produit davantage amplifié en utilisant la présence ou l'absence d'une unité distinctive introduite par hybridation ou appariement d'un composant, lequel comprend l'unité distinctive, dans la séquence complémentaire à l'autre partie.

2. Procédé selon la revendication 1 dans lequel deux amorces ou plus du second ensemble d'amorces comprennent une seconde autre partie, la seconde autre partie de l'une des amorces comprenant une séquence qui correspond à la séquence d'au moins une partie de l'autre partie de l'une des amorces du premier ensemble, la seconde autre partie de l'une des amorces comprenant une séquence qui correspond à la séquence d'au moins une partie de l'autre partie d'une amorce différente parmi les amorces du premier ensemble.

3. Procédé selon la revendication 1 ou la revendication 2 dans lequel l'unité distinctive est introduite pendant le procédé d'amplification.

4. Procédé selon la revendication 1 ou la revendication 2 dans lequel l'unité distinctive est introduite dans une étape qui est ultérieure au procédé d'amplification.

5. Procédé selon la revendication 4 dans lequel le composant est une sonde pour l'autre partie.

6. Procédé selon l'une quelconque des revendications précédentes dans lequel les premiers ensembles d'amorces sont composés de deux amorces sens et d'une amorce anti-sens.

7. Procédé selon l'une quelconque des revendications précédentes dans lequel l'autre partie du premier ensemble d'amorces est fixée à l'extrémité 5' de la partie spécifique d'un locus.

8. Procédé selon l'une quelconque des revendications précédentes dans lequel l'extrémité 3' des amorces sens des premiers ensembles d'amorces est munie d'une partie identifiant un SNP.

9. Procédé selon la revendication 8 dans lequel la partie spécifique d'un locus et la partie identifiant un SNP de l'une des amorces sens s'apparient avec le côté 3' du locus possédant le SNP en cours d'étude, et la partie spécifique d'un locus et la partie identifiant un SNP de l'autre amorce sens de ce premier ensemble ne s'apparient pas avec le côté 3' du SNP en cours d'étude,

10. Procédé selon la revendication 8 dans lequel l'amorce s'appariant s'apparie en raison d'une correspondance entre la partie identifiant un SNP et le site SNP de l'échantillon, et l'amorce ne s'appariant pas ne s'apparie pas en raison d'un mésappariement entre la partie identifiant un SNP et le site SNP de l'échantillon.

11. Procédé selon l'une quelconque des revendications précédentes dans lequel un seul second ensemble d'amorces est prévu.

12. Procédé selon l'une quelconque des revendications précédentes dans lequel le second ensemble d'amorces est composé de deux amorces sens et d'une amorce anti-sens.

13. Procédé selon l'une quelconque des revendications précédentes dans lequel la seconde autre partie comprend une séquence qui correspond à la séquence de la première autre partie et/ou qui s'apparie avec la séquence du produit amplifié correspondant à la première autre partie.

14. Procédé selon l'une quelconque des revendications précédentes dans lequel une pluralité de premiers ensembles d'amorces est prévue pour amplifier une pluralité de loci SNP, les produits d'amplification obtenus étant de longueurs différentes.

15. Procédé selon l'une quelconque des revendications précédentes dans lequel un premier ensemble de conditions d'amplification et un second ensemble de conditions d'amplification sont utilisés, le premier ensemble de conditions d'amplification inhibant l'amplification par une ou plusieurs des amorces.

16. Procédé selon la revendication 15 dans lequel le second ensemble de conditions d'amplification permet l'amplification de la ou des amorce(s) précédemment inhibée(s).

17. Procédé selon l'une quelconque des revendications précédentes dans lequel le premier et le second ensembles d'amorces sont présents ensemble et la température d'appariement pour au moins certains des cycles du processus d'amplification est telle qu'au moins 80 % du second ensemble d'amorces demeurent simple brin.

18. Procédé selon la revendication 17 dans lequel la température d'appariement est prévue et utilisée ainsi au moins dans les cycles 3 à 30.

19. Procédé selon l'une quelconque des revendications précédentes dans lequel une température d'appariement est utilisée dans au moins les deux derniers cycles, la température d'appariement permettant qu'au moins 80 % du second ensemble d'amorces s'apparient.

20. Procédé selon l'une quelconque des revendications précédentes dans lequel la température d'appariement est au moins de 72 °C pour les cycles 3 à 30 du processus d'amplification.

21. Procédé selon l'une quelconque des revendications précédentes dans lequel la température d'appariement pour au moins les deux derniers cycles du processus d'amplification est de 62 °C ou moins.

22. Procédé selon l'une quelconque des revendications précédentes dans lequel le premier et le second ensembles d'amorces sont présents ensemble et dans lequel la concentration du second ensemble d'amorces est prévue selon un rapport relativement à la concentration du premier ensemble d'amorces d'au moins 5/1.

23. Procédé selon l'une quelconque des revendications précédentes dans lequel le premier et le second ensembles d'amorces sont présents ensemble, le premier ensemble d'amorces étant prévu à une concentration se trouvant entre 10 et 200 nM et le second ensemble étant prévu à une concentration se trouvant entre 400 et 4000 nM.

24. Procédé selon l'une quelconque des revendications précédentes dans lequel l'unité distinctive est un colorant, un marqueur coloré, une molécule chromogène, un phare moléculaire, un émetteur de rayonnements, un isotope caractéristique.

25. Procédé selon la revendication 3 ou l'une quelconque des revendications en dépendant dans lequel une unité distinctive est introduite pendant l'amplification.

26. Procédé selon la revendication 3 ou l'une quelconque des revendications en dépendant dans lequel une unité distinctive est prévue à l'extrémité 5' des amorces sens du second ensemble d'amorces, une unité distinctive différente étant prévue pour chaque amorce sens dans un second ensemble.

27. Procédé selon l'une quelconque des revendications précédentes dans lequel l'unité distinctive est indicative de la présence d'un nucléotide au niveau du SNP.

28. Procédé selon l'une quelconque des revendications précédentes dans lequel les produits d'amplification de deux premiers ensembles d'amorces ou plus et d'un ou plusieurs seconds ensembles d'amorces sont séparés les uns des autres par électrophorèse.

29. Procédé selon la revendication 4 ou l'une quelconque des revendications en dépendant dans lequel le produit davantage amplifié comprend une unité de fixation et l'unité de fixation facilite la fixation du produit davantage amplifié à un support solide.

30. Procédé selon la revendication 29 dans lequel le produit davantage amplifié fixé est mis en contact avec une ou plusieurs sondes ayant des séquences différentes les unes des autres, au moins en partie, le contact aboutissant à l'hybridation d'une des sondes avec le produit davantage amplifié.

31. Procédé selon la revendication 29 ou la revendication 30 dans lequel chaque sonde a une partie de séquence commune entre elles, la partie de séquence commune correspondant en séquence à la partie spécifique d'un locus du produit davantage amplifié.

32. Procédé selon l'une quelconque des revendications 29 à 31 dans lequel les sondes intègrent au moins une partie de séquence différente les unes par rapport aux autres, la partie de séquence différente d'au moins une des sondes correspondant à la séquence de l'autre partie qui est comprise dans la séquence du produit davantage amplifié.

33. Procédé selon l'une quelconque des revendications 29 à 32 dans lequel le contact des sondes avec le produit davantage amplifié aboutit à l'hybridation de l'une des sondes avec le produit davantage amplifié, chaque sonde ayant une unité distinctive par rapport à l'autre.

34. Procédé selon l'une quelconque des revendications précédentes dans lequel une ou plusieurs amorce(s) du premier ensemble d'amorces est ou sont dotée(s) d'une partie identifiant un SNP, la partie identifiant un SNP étant différente pour chaque amorce différente, l'amorce possédant une partie d'identité avec le SNP qui s'apparie avec le SNP s'hybridant avec un côté du SNP.

35. Procédé selon l'une quelconque des revendications précédentes dans lequel la partie spécifique d'un locus des amorces du premier ensemble comprend une séquence qui correspond à la séquence de la séquence du locus dans le voisinage du SNP en cours d'étude, la correspondance entre la partie spécifique d'un locus et la séquence du locus commençant à un niveau situé entre une et dix bases sur les côtés respectifs du SNP en cours d'étude.

36. Procédé selon l'une quelconque des revendications précédentes dans lequel le premier ensemble d'amorces comprend une amorce anti-sens et comprend en outre une amorce sens pour chaque identité possible du SNP en cours d'étude.

37. Procédé selon l'une quelconque des revendications précédentes dans lequel la partie spécifique d'un locus des amorces dans un ensemble est dotée de séquences identiques dans chaque amorce.

38. Procédé selon l'une quelconque des revendications précédentes dans lequel l'autre partie comprend une séquence qui ne correspond pas à la séquence du locus sur le côté 3' du locus auquel la partie spécifique d'un locus de l'amorce correspond.

39. Procédé selon l'une quelconque des revendications précédentes dans lequel le produit davantage amplifié est mis en contact avec un ou plusieurs composants retenu(s) sur un support solide, le un ou plusieurs composant(s) possédant une séquence qui s'apparie avec au moins une partie de la séquence de l'un des produits davantage amplifiés.

40. Procédé selon la revendication 39 dans lequel le composant retenu s'apparie avec le produit davantage amplifié jusqu'à la base précédant la base qui est le côté du SNP.

41. Procédé selon la revendication 39 dans lequel le composant retenu s'apparie avec le produit davantage amplifié le long de la séquence correspondant à la partie spécifique d'un locus et à l'autre partie du produit davantage amplifié.

42. Procédé selon l'une quelconque des revendications 39 à 41 dans lequel une pluralité de composants retenus différents, de préférence des produits de PCR et/ou des oligonucléotides, sont prévus au niveau d'emplacements distincts sur un support, des composants retenus différents s'appariant avec des produits davantage amplifiés différents.

43. Procédé selon la revendication 41 dans lequel le composant retenu et le produit davantage amplifié apparié sont mis en contact avec un ou plusieurs autre(s) composant(s) pour introduire une unité distinctive.

44. Procédé selon la revendication 41 dans lequel le composant retenu et le produit davantage amplifié apparié sont mis en contact avec un ou plusieurs composant(s) supplémentaire(s), le ou les composant(s) supplémentaire(s) étant un ou plusieurs autre(s) oligonucléotide(s) qui comprend ou comprennent une unité distinctive.

45. Procédé selon la revendication 44 dans lequel la base terminale de l'autre oligonucléotide est l'une des quatre identités possibles pour le SNP.
